## Europäisches Patentamt
## European Patent Office
## Office européen des brevets

(19)

(11) **EP 0 513 336 B1**

(12)

# FASCICULE DE BREVET EUROPEEN

(45) Date de publication et mention
de la délivrance du brevet:
**12.11.2003 Bulletin 2003/46**

(21) Numéro de dépôt: **92902218.4**

(22) Date de dépôt: **29.11.1991**

(51) Int Cl.7: **C12N 15/63**, C12N 15/81,
C12P 1/02, C12N 1/16,
A61K 38/21

(86) Numéro de dépôt international:
**PCT/FR91/00953**

(87) Numéro de publication internationale:
**WO 92/009691 (11.06.1992 Gazette 1992/13)**

(54) **NOUVEAUX VARIANTS DERIVES DES INTERFERONS DE TYPE I, LEUR PROCEDE DE PRODUCTION, ET LEURS APPLICATIONS**

NEUE TYPE-I-INTERFERON VARIANTEN, IHRE VERFAHREN ZUR HERSTELLUNG, UND IHRE VERWENDUNGEN

NEW VARIANTS DERIVED FROM INTERFERONS OF TYPE I, PRODUCTION PROCESS THEREOF AND THEIR APPLICATIONS

(84) Etats contractants désignés:
**AT BE CH DE DK ES FR GB GR IT LI LU NL SE**

(30) Priorité: **29.11.1990 FR 9014945**
**29.11.1990 FR 9014946**

(43) Date de publication de la demande:
**19.11.1992 Bulletin 1992/47**

(73) Titulaire: **INSTITUT NATIONAL DE LA
RECHERCHE AGRONOMIQUE (INRA)
75341 Paris Cédéx 07 (FR)**

(72) Inventeurs:
• **MARTAL, Jacques
F-78350 Jouy-en-Josas (FR)**
• **DEGRYSE, Eric
F-67100 Strasbourg (FR)**
• **GAYE, Pierre
F-75018 Paris (FR)**
• **CHARLIER, Madia
F-75014 Paris (FR)**
• **CHARPIGNY, Gilles
F-45000 Orléans (FR)**
• **REINAUD, Pierrette
F-92320 Châtillon (FR)**

• **CHAOUAT, Gérard
F-75020 Paris (FR)**

(74) Mandataire: **Ores, Irène et al
Cabinet ORES
36,rue de St Pétersbourg
75008 Paris (FR)**

(56) Documents cités:
| | |
|---|---|
| **EP-A- 0 088 632** | **EP-A- 0 123 544** |
| **EP-A- 0 220 958** | **WO-A-84/00776** |
| **WO-A-89/08706** | **WO-A-90/09806** |
| **WO-A-90/13646** | |

• **Gene, vol. 22, 1983, (Amsterdam, NL), I. PALVA et al.: "Secretion of interferon by Bacillus subtilis", pages 229-235, voir l'article en entier surtout page 231, colonne de droite, lignes 30-36; page 233, figure 2; page 234**
• **The Journal of Biological Chemistry, vol. 261, no. 12, 5 mai 1986, (Baltimore, US), K.M. ZSEBO et al.: "Protein secretion from Saccharomyces cerevisiae directed by the prepro-alpha-factor leader region", pages 5858-5865, voir l'article en entier (citée dans de la demande)**

EP 0 513 336 B1

**Description**

La présente Invention concerne des nouveaux variants dérivés des interférons de type I, obtenus par les techniques de l'ADN recombinant à partir de la levure.

Les interférons ont été identifiés à l'origine par leur capacité d'empêcher la réplication virale. Ils constituent une famille de protéines qui ont été réparties en différents groupes. La classification actuellement proposée pour les interférons distingue deux types principaux :

-   le type I qui regroupe les interférons α, ω, β, et les trophoblastines ;
-   et le type II qui comprend les interférons γ.

Les interférons α et ω étaient précédemment connus sous les dénominations respectives d'interféron α-I (α de classe I) et α-II (α de classe II) et les trophoblastines auraient pu être classées parmi les interférons α-III.

Outre leur activité antivirale, les interférons peuvent posséder d'autres fonctions. Par exemple, il a déjà été établi que, chez certains mammifères tels que les bovins et les ovins, la trophoblastine intervenait dans le phénomène de reconnaissance maternelle de la gestation.

La trophoblastine ovine ou oTP, a été mis en évidence respectivement par MARTAL et al. [J. Reprod. Fert., 56, 63-73 (1979) ; Pro. 10th intern. Congress on Anim. Reprod. and A.I., Urbana-Champaign (USA), 11, 509 (short communication) (1984)] et GODKIN et al. [J. Reprod. Fert., 65, 141-150 (1982)] chez les ovins où elle est produite en abondance par l'embryon, entre le 12e et le 21e jour de gestation ; chez les bovins, elle est produite entre le 16ème et le 24ème jour. Elle existe sous au moins 5 isoformes correspondant à différents allèles. Ces isoformes sont décrites dans la Demande Internationale PCT publiée sous n' WO 89/08706, au nom de l'INSTITUT NATIONAL DE LA RECHERCHE AGRONOMIQUE. Le poids moléculaire de la trophoblastine est de 20 KDa et son point isoélectrique est compris entre 5,3 et 5,5, suivant l'isoforme considérée. C'est chez certains ruminants que les trophoblastines ont été le mieux caractérisées. Toutefois, des travaux récents montrent que des trophoblastines-like existeraient également chez d'autres mammifères (cheval, lapin) et chez l'homme.

La trophoblastine produite par les embryons possède, comme les autres interférons, des activités biologiques variées.

La trophoblastine intervient en particulier dans le mécanisme de reconnaissance de l'embryon par l'organisme maternel.

Dans la plupart des cas, la durée d'une gestation dépasse largement celle de la phase lutéale du cycle ovarien. Lorsqu'il y a eu fécondation, certains mécanismes interviennent afin de prolonger la durée de vie du corps jaune et d'empêcher un retour du cycle ovarien. Chez les ruminants, l'embryon émet un signal biochimique sous forme de trophoblastine (oTP). Cette substance permet à l'organisme de continuer de sécréter de la progestérone, une hormone qui est essentielle pour un développement embryonnaire normal.

GODKIN et al. [J. Reprod. Fert., 71, 57-64 (1984)] ont montré que l'injection intra-utérine d'oTP purifiée prolonge pendant quelques jours la sécrétion de progestérone lutéale chez des brebis cycliques receveuses. FINCHER et al. [J. Reprod. Fert., 76, 425-433 (1986)] ont constaté que l'injection d'oTP naturelle dans l'utérus retarde de quelques jours la lutéolyse induite par l'ocytocine ou l'oestradiol et s'accompagne d'une réduction de la sécrétion de prostaglandine $F_{2\alpha}$. Des observations similaires ont été faites avec des interférons α recombinants de classe I [PLANTE et al., Endocrinology 122, 2342-2344, (1988) ; STEWART et al., (1989) J. Repr. Fert. Supp. p. 127-138].

La trophoblastine n'est sécrétée par l'embryon que pendant une période relativement courte : du 16e au 24e jour de gestation en ce qui concerne les bovins et du 12e au 22e jour de gestation dans le cas du mouton.

Si la mère et l'embryon sont asynchrones, c'est-à-dire si l'embryon sécrète la trophoblastine avant que la mère soit physiologiquement capable d'y être sensible et de répondre à une telle stimulation, le corps jaune régresse et l'embryon meurt. Cette éventualité est particulièrement critique lorsqu'il s'agit de transfert d'embryon. A ce jour, le taux d'échec lors des transferts d'embryons est supérieur à 30%. D'un point de vue économique, ceci est particulièrement désastreux, ce qui est encore plus accusé lors de fécondation *in vitro*.

Un grand nombre de mortalité embryonnaire serait due au fait que l'état de développement de l'embryon et les conditions physiologiques maternelles ne seraient pas "en phase" au moment de l'implantation de l'embryon dans l'utérus de la mère porteuse. De plus, les embryons sont souvent congelés avant transfert avec, pour conséquence, la perte d'une certaine capacité de production de trophoblastine produite par les embryons.

Une activité antivirale de la trophoblastine a également été mise en évidence, par exemple par MARTAL et al. [J.Reprod.Fert.Abs.series, 2, 3, (1988)] et PONTZER et al., [Biochem.Biophys.Res.Com., 152, 801-807, (1988)], ainsi que dans la Demande PCT 89/08706 précitée. Cette Demande propose également l'utilisation de la trophoblastine pour la prévention du rejet des greffes d'organes.

Ces propriétés permettent d'envisager de nombreuses utilisations thérapeutiques de la trophoblastine. Le développement de telles applications s'est heurté toutefois au fait que la trophoblastine ne pouvait être obtenue qu'à partir des conceptus, ce qui ne permettait pas sa production en quantité suffisante pour un usage *in-vivo*.

Compte tenu de ce qui précède, il serait donc très intéressant de disposer de grandes quantités de trophoblastine afin de pouvoir traiter les animaux en début de gestation. Seules les techniques de l'ADN recombinant peuvent permettre d'atteindre ce but.

Le clonage chez E. Coli de l'ADN complémentaire (ADNc) des ARN messagers (ARNm) codant pour la trophoblastine ovine a été décrit dans la Demande PCT 89/08706 citée plus haut. Il pouvait donc être envisagé de construire, à partir de cet ADNc, des gènes chimériques permettant la production de trophoblastine dans des microorganismes.

Néanmoins, les premiers essais d'expression d'un ADNc codant pour la trophoblastine ovine ne se sont pas révélés concluants. Ces premiers essais mettaient en oeuvre des bactéries *(E. coli)* ou des levures (*S. cerevisiae*). En particulier une cassette d'expression destinée à une production de trophoblastine ovine dans la levure et comprenant un fragment d'ADN codant pour un peptide signal associé à l'extrémité 5' de l'ADNc codant pour la trophoblastine ovine mature n'a pas permis d'obtenir une synthèse à un taux convenable.

D'autre part, ZSEBO et al., [J. Biol. Chem. 261 (13) : 5858, (1986)] rapportent qu'un interféron α ne peut être sécrété dans de bonnes conditions et en grande quantité en utilisant le système "pré-pro" du facteur-α.

En outre, d'une manière générale, les interférons de type I sont caractérisés par la présence de quatre résidus cystéine en positions 1, 29, 99, et 139 qui s'associent entre eux pour former les ponts disulfure cys1-cys99 et cys29-cys139, et il est considéré que le résidu cystéine en position N-terminale est essentiel pour le maintien d'une structure conformationnelle correcte.

De manière surprenante, les inventeurs ont maintenant trouvé que l'addition d'un fragment d'ADN codant pour un acide aminé complémentaire, ou pour un di- ou tripeptide, tel que par exemple pour le dipeptide Ala-Pro, ou Ala-Gly, à l'extrémité 5' de l'ADNc d'un interféron de classe trophoblastine possédant une cystéine en position N-terminale était bénéfique. En effet, l'ensemble de la structure codante est convenablement exprimée dans la levure, et les variants ainsi produits sont sécrétés en quantité plus importante que ceux codés par une structure dépourvu de ce fragment. De plus, il a pu être établi que les variants obtenus de la sorte conservent une activité biologique similaire à celle des trophoblastines naturelles. Par exemple, l'addition d'un dipeptide à l'extrémité N-terminale de la trophoblastine ovine donne lieu à des variants qui conservent les activités antivirales, immunologiques et anti-lutéolytique de la trophoblastine naturelle.

En conséquence, l'invention propose de nouveaux variants d'interféron, répondant à l'une des formules suivantes :

$$X_1\text{-}R_0 \qquad\qquad (I)$$

ou bien

$$X_1\text{-}X_2\text{-}R_0 \qquad\qquad (II)$$

ou bien

$$X_1\text{-}X_2\text{-}X_3\text{-}R_o \qquad\qquad (III)$$

dans laquelle $X_1$, $X_2$ et $X_3$ sont identiques ou différents, et représentent chacun un acide aminé, et $R_0$ représente la séquence en acides aminés de la forme mature d'un interféron de classe trophoblastine.

Préférentiellement, $X_1$, $X_2$ et $X_3$ représentent chacun un acide aminé acide, un acide aminé basique, ou un acide aminé choisi dans le groupe constitué par l'alanine, la valine, la proline, la glycine, la sérine, la thréonine, la cystéine, l'asparagine, la glutamine, et $X_1$ représente un acide aminé différent de la proline.

De manière avantageuse, un variant selon l'Invention répond à l'une des formules :

$$Ala\text{-}Pro\text{-}R_0' \qquad\qquad (IV)$$

ou

$$\text{Ala-Gly-}R_0' \tag{V}$$

dans laquelle $R_0'$ représente la séquence en acides aminés de la forme mature d'une trophoblastine. De préférence, un variant selon l'Invention répond à l'une des formules :

$$\text{Ala-Pro-}R_0'' \tag{VI}$$

ou

$$\text{Ala-Gly-}R_0'' \tag{VII}$$

dans laquelle $R_0''$ représente la séquence en acides aminés de la forme mature d'une trophoblastine d'origine bovine ou ovine.

De manière tout à fait préférée, un variant selon l'Invention est de formule :

$$\text{Ala-Pro-}R_0''' \tag{VIII}$$

ou

$$\text{Ala-Gly-}R_0''' \tag{IX}$$

dans laquelle $R_0'''$ représente la séquence en acides aminés de la forme mature d'une quelconque des isoformes de la trophoblastine ovine.

Des isoformes de la trophoblastine ovine, respectivement dénommées T1, T2, T3, T4, et T5 sont décrites dans la Demande PCT 89/08706.

Dans le cadre de la présente Invention, les variants préférés de formules (VIII) ou (IX) incluent des variants dans lesquels $R_0'''$ représente la séquence en acides aminés de la forme mature de l'une quelconque des isoformes T1 à T5 de la trophoblastine ovine.

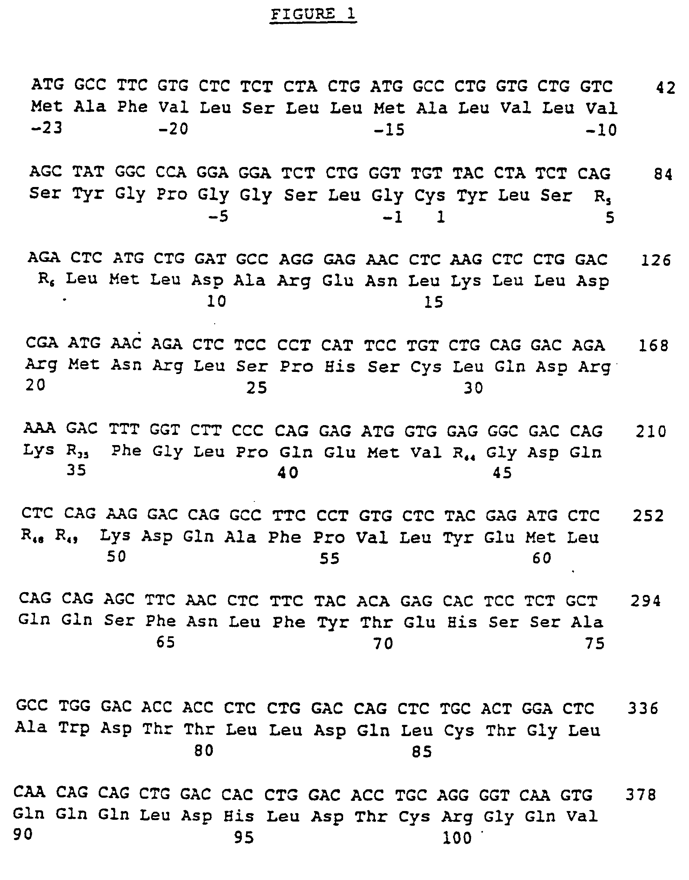

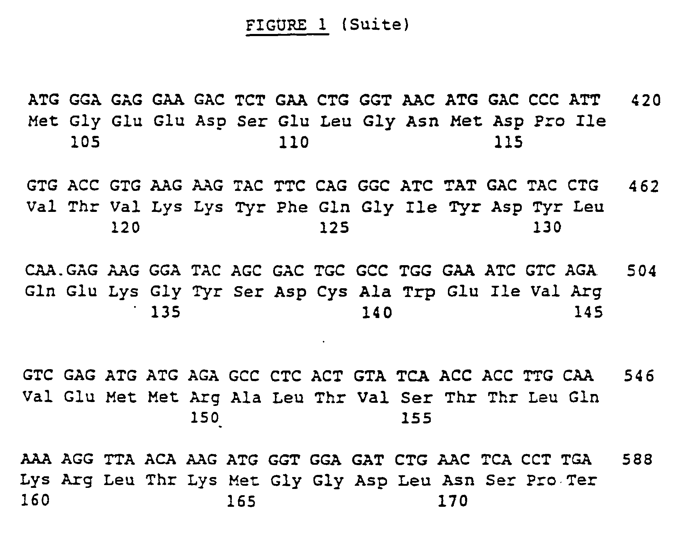

La figure 1 donne en exemple la séquence en acides aminés d'isoformes de la trophoblastine, commençant avec le résidu cystéine en position 1 et finissant avec le résidu proline en position 172 (peptide signal de -23 à -1), dans laquelle :

.   $R_5$ est un résidu acide glutamique, glutamine ou arginine,
.   $R_6$ est un résidu arginine ou lysine,
.   $R_{35}$ est un résidu lysine ou acide aspartique, et
.   $R_{44}$ est un résidu acide glutamique ou acide aspartique.
.   $R_{48}$ est un résidu leucine ou acide aspartique.
.   $R_{49}$ est un résidu leucine ou glutamine.

Des variants préférés entrant dans la définition des formules (VIII) ou (IX) incluent:

-   un variant de formule (Xa) dans laquelle $R_5$ est un résidu arginine, $R_6$ est un résidu lysine, $R_{35}$ est un résidu acide aspartique, $R_{44}$ est un résidu acide glutamique, $R_{48}$ est un résidu leucine, et $R_{49}$ un résidu glutamine,
-   un variant de formule (Xb) dans laquelle $R_5$ est un résidu acide glutamique, $R_6$ est un résidu arginine, $R_{35}$ est un résidu lysine, $R_{44}$ est un résidu acide glutamique, $R_{48}$ est un résidu acide aspartique, et $R_{49}$ un résidu leucine,
-   un variant de formule (Xc) dans laquelle $R_5$ est un résidu glutamine, $R_6$ est un résidu arginine, $R_{35}$ est un résidu acide aspartique, et $R_{44}$ est un résidu acide aspartique, et,
-   un variant de formule (Xd) dans laquelle $R_5$ est un résidu glutamine, $R_6$ est un résidu arginine, $R_{35}$ est un résidu acide aspartique, $R_{44}$ est un résidu acide glutamique, $R_{48}$ est un résidu leucine, et $R_{49}$ un résidu glutamine.

D'autre part, l'Invention a aussi pour objet une cassette d'expression d'un variant selon l'Invention qui comprend au moins :

- un premier fragment d'ADN codant pour un variant selon l'Invention,
- un deuxième fragment d'ADN codant pour un peptide signal, ledit deuxième fragment d'ADN étant associé à l'extrémité 5' du premier fragment d'ADN,
- un promoteur permettant l'expression desdits fragments d'ADN dans la levure.

Une telle cassette est capable de promouvoir l'expression d'un précurseur peptidique constitué du côté N-terminal, d'un peptide signal et, du côté C-terminal, d'un variant selon l'invention. Lors du passage dans le réticulum endoplasmique le peptide signal sera éliminé par clivage pour libérer le variant sous forme mature.

Pour l'usage dans une cassette d'expression selon l'Invention, ledit deuxième fragment d'ADN est tel qu'il peut coder pour n'importe quel peptide signal dont l'extrémité C-terminale constitue un site de protéolyse capable d'être reconnu par une signal-peptidase de l'organisme hôte destiné à l'hébergement de la cassette d'expression. La signal-peptidase doit nécessairement couper l'extrémité C-terminale du peptide signal.

Dans le cadre de la présente Invention, des peptides signal avantageux incluent par exemple :

- le peptide signal du précurseur du facteur-α ayant la séquence d'acides aminés Met-Arg-Phe-Pro-Ser-Ile-Phe-Thr-Ala-Val-Leu-Phe-Ala-Ala-Ser-Ser-Ala-Leu-Ala (le site de protéolyse est souligné) ;
- le peptide signal du précurseur de la β-1,-3 glucanase de levure ayant la séquence d'acides aminés Met-Arg-Phe-Ser-Thr-Thr-Leu-Ala-Thr-Ala-Ala-Thr-Ala-Leu-Phe-Phe-Thr-Ala-Ser-Gln-Val-Ser-Ala (le site de protéolyse est souligné) ;

ainsi que les dérivés fonctionnels de ceux-ci.

Par exemple, des dérivés fonctionnels du peptide signal du précurseur de la β-1,-3 glucanase sont décrits dans la demande PCT N° de Dépôt FR90/00306 du 27.04.90.

D'une manière générale, le promoteur destiné à l'expression du précurseur d'un variant selon l'invention peut être n'importe quel promoteur fonctionnel dans la levure, de préférence un promoteur capable d'induire un bon niveau d'expression d'une séquence codante quelconque. Un promoteur avantageux est, par exemple, le promoteur du gène MFα1 qui code pour le facteur α ou un dérivé fonctionnel de celui-ci.

Enfin, l'Invention a aussi pour objet :

- une cellule de levure transformée par une cassette d'expression selon l'invention et,
- un procédé de production d'un variant selon l'invention, qui comprend l'acte de cultiver une cellule de levure transformée par une cassette d'expression selon l'invention et de récolter ledit variant à partir du surnageant de culture.

La cassette d'expression selon l'invention telle que présente dans la cellule de levure transformée, peut être soit incorporée dans le génome de la levure, soit être portée par un plasmide capable de se répliquer dans la levure. Dans ce dernier cas, il convient de choisir un couple plasmide:levure-hôte tel que le plasmide puisse être maintenu dans la levure par pression de sélection. Avantageusement, on choisit d'une part, une levure-hôte auxotrophe pour un métabolite indispensable à la croissance cellulaire et d'autre part, un plasmide qui permet de complémenter cette auxotrophie.

Les variants de trophoblastine selon l'Invention (ces variants sont ci-après désignés par le terme général APrT) sont utilisables dans toutes les applications des interférons naturels telles que par exemple l'obtention de médicaments antiviraux, immunomodulateurs, anti-inflammatoires, antitumoraux.

En outre, ils peuvent être plus spécifiquement utilisés pour l'obtention de médicaments antilutéolytiques, ainsi que de produits destinés à améliorer la survie des embryons lors de leur transplantation, et pour l'obtention de réactifs permettant le diagnostic de la viabilité des embryons à un stade précoce de leur développement. Ils sont également utilisables comme immunogènes pour induire la production d'anticorps anti-trophoblastine.

Selon un mode de réalisation préféré de la présente Invention, l'APrT est utilisée pour le traitement des troupeaux, afin d'améliorer leur fertilité.

Selon un autre mode de réalisation préféré de la présente Invention, l'APrT est utilisée pour le traitement des embryons lors de leur transplantation, dans les différentes techniques de reproduction d'animaux d'élevage impliquant un transfert d'embryons, en particulier celles associées à la cryoconservation, à la fécondation in-vitro, au clonage embryonnaire, à la transgénèse embryonnaire.

Selon encore un autre mode de réalisation préféré de la présente Invention, l'APrT est utilisée pour l'obtention de réactifs et de kits permettant le diagnostic de viabilité des embryons à un stade précoce de leur développement.

Un tel diagnostic est basé sur le dosage de la trophoblastine produite par les embryons. Ce dosage peut par exemple, être effectué par des méthodes immunologiques ; dans ce cas, l'APrT peut être utilisé comme immunogène pour la production d'anticorps, ou comme antigène, dans des méthodes de type compétitif. La trophoblastine produite par les embryons peut également être quantifiée par son activité antivirale ; l'APrT peut être utilisée comme standard d'activité antivirale dans un tel dosage.

Les propriétés immunologiques de l'APrT peuvent également être mises à profit pour induire, chez un mammifère dont on souhaite provoquer l'infertilité, l'apparition d'anticorps anti-trophoblastine.

La présente Invention a également pour objet un procédé de purification de l'APrT à partir de milieu de culture de levures la produisant, lequel procédé est caractérisé en ce que l'APrT est purifiée par chromatographie (par exemple de type DEAE) sur une colonne échangeuse d'ions anionique, par une élution en 3 étapes :

- un gradient de KCl de 0 à 0,135 M
- une phase isocratique à 0,135 M environ de KCl
- un gradient de KCl de 0,135 M à 0,5 M,

l'APrT étant recueillie entre 0,135 et 0,3 M de KCl.

L'APrT peut également être purifiée par chromatographie en phase inverse, ou par chromatographie d'affinité, à l'aide d'anticorps anti-trophoblastine.

La présente Invention sera mieux comprise à l'aide du complément de description qui va suivre, qui se réfère à des exemples de préparation de variants d'interféron de type I conformes à l'Invention.

Il va de soi toutefois que ces exemples sont donnés uniquement à titre d'illustration de l'objet de l'Invention, dont ils ne constituent en aucune manière une limitation.

**EXEMPLE 1 : Construction du plasmide d'expression du variant Ala-Pro de la trophoblastine ovine dans la levure (pTG7908)**

La séquence d'ADN codant pour le précurseur de la trophoblastine ovine telle que décrite dans la Demande PCT WO 89/08706 (voir aussi figure 1) est clonée sous forme d'un fragment EcoRI dans le vecteur M13TG131 décrit dans l'article de M.P. KIENY et al., Gene (1983) 26:91. On obtient ainsi le vecteur M13TG771. Pour pouvoir isoler le fragment codant pour la protéine mature, c'est-à-dire le fragment d'ADN sans la séquence signal, on crée, en 5' de la séquence mature de la protéine, un site HindIII par mutagénèse dirigée en utilisant la trousse AMERSHAM et l'oligonucléotide OTG2102 dont la séquence est la suivante : GAGGATCTCAAGCTTGTTACCTAT.

Le brin antisens de la trophoblastine, porté par le vecteur M13TG771 simple brin, est représenté ci-dessous ligne I et l'oligonucléotide OTG2102 est représenté ligne II ; les étoiles (*) représentent les mésappariements qui vont provoquer les mutations recherchées.

```
Ligne I   : CT CCT AGA GAC CCA ACA ATG GAT A
Ligne II  : GA GGA TCT CAA GCT TGT TAC CTA T.
                       ** *
```

On obtient ainsi le vecteur M13TG7720. Les mutations ponctuelles qui ont été introduites dans la séquence codante induisent le remplacement des acides aminés Leucine en position -2 et Glycine en position -1 du précurseur de la trophoblastine, respectivement par la glutamine et l'alanine. M13TG7720 est ensuite digéré par EcoRI et le fragment d'ADN EcoRI codant pour le précurseur muté de la trophoblastine est inséré dans le vecteur pTG769 (décrit dans la Demande de Brevet EP 0258118) préalablement digéré par EcoRI. On obtient ainsi le vecteur pTG7901.

D'autre part, pour produire la trophoblastine dans la levure, le fragment d'ADN codant pour la trophoblastine doit être mis sous le contrôle d'un promoteur de levure. Pour cela on utilise le vecteur M13TG3841, décrit dans la Demande de Brevet PCT déposée le 28 Avril 1990, dont le numéro de dépôt est FR90/00306, et qui contient en particulier :

- le promoteur du gène codant pour le facteur alpha 1 (MFalpha1) ;
- la séquence pré de MFalpha1 codant pour le peptide signal du précurseur du facteur alpha 1 ; et
- la séquence pro de MFalpha1.

Un site de restriction SmaI est créé entre le deuxième et le troisième codon de la séquence pro de MFalpha1 par mutagénèse dirigée en utilisant la trousse Amersham et l'oligonucléotide OTG2072 dont la séquence est la suivante : TCCGCATTAGCTGCTCCCGGGAACACTACAACAGAA.

Le brin antisens des séquences pré-pro de MFalpha1, porté par le vecteur M13TG3841, est représenté ci-dessus ligne I et l'oligonucléotide OTG2072 est représenté ligne II ; les étoiles (*) indiquent les mésappariements qui vont provoquer les mutations recherchées.

```
Ligne I    AGG CGT AAT CGA CGA GGT CAG TTG TGA TGT TGT CTT
Ligne II   TCC GCA TTA GCT GCT CCC GGG AAC ACT ACA ACA GAA
                                    *   **
                                   Sma I
```

On obtient ainsi le vecteur M13TG3869. Les mutations ponctuelles qui ont été introduites dans la séquence codante induisent le remplacement de la glycine par la valine.

Le vecteur pTG7901 est digéré par HindIII pour libérer le fragment d'ADN HindIII, codant pour la trophoblastine mature qui, est ensuite traité à la nucléase Mung Bean. Ce fragment est inséré dans le vecteur M13TG3869 préalablement digéré par SmaI. On obtient ainsi le vecteur M13TG7740 qui comporte en séquence et en phase :

- le promoteur de MFalpha1,
- la séquence pré de MFalpha1 suivie des deux premiers codons de la séquence pro, soit ceux codant pour les acides aminés alanine et proline et,
- le fragment d'ADN codant pour la trophoblastine ovine.

Le fragment d'ADN SpHI, issu du vecteur M13TG7740, comportant le promoteur de MFalpha1, la séquence pré suivie des codons de l'alanine et de la proline et la séquence d'ADN codant pour la trophoblastine mature est inséré dans le vecteur de levure pTG3828 (décrit dans la Demande de Brevet PCT dont le numéro de dépôt est FR90/00306) préalablement digéré par SpHI. On obtient ainsi le plasmide pTG7908.

En procédant à la mutagénèse dirigée du vecteur M13TG7740 en utilisant la trousse Amersham comme décrit ci-dessus et l'oligonucléotide OTG2643, on obtient le remplacement de la séquence codant pour l'extension N-terminale Ala-Pro par une séquence codant pour Ala-Gly. Un fragment SpHI du vecteur M13TG7745 obtenu de la sorte est inséré, comme décrit ci-dessus, dans le plasmide pTG3828 ; le plasmide résultant est dénommé pTG7941.

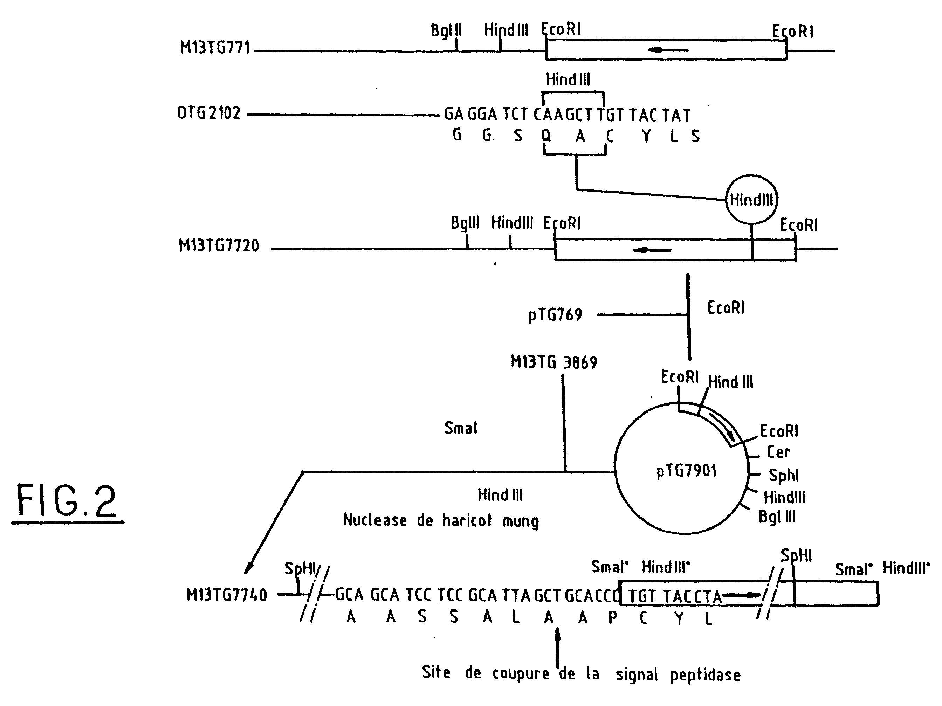

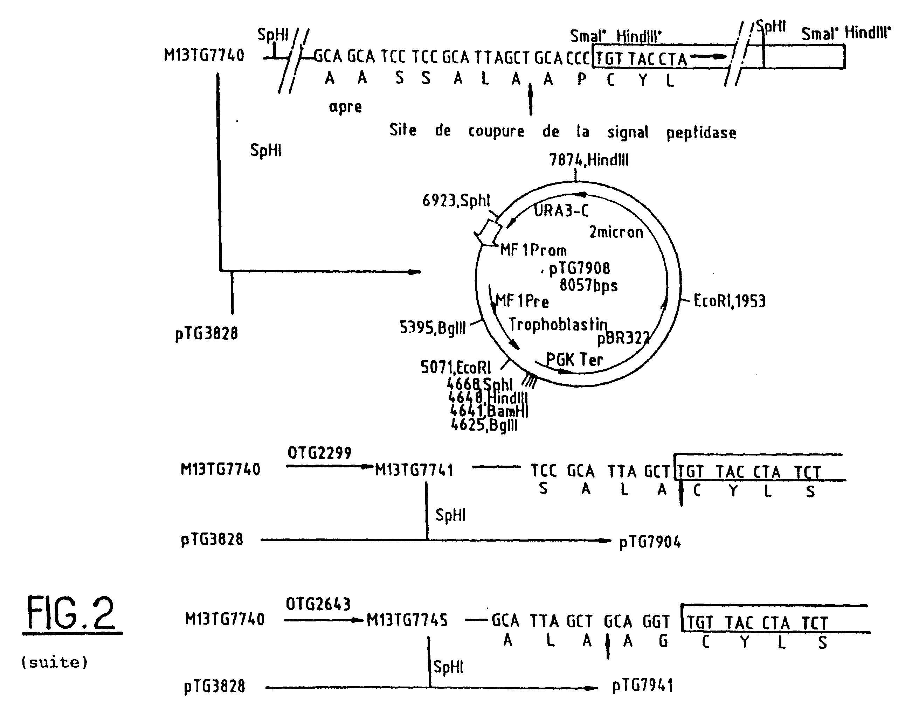

Un plasmide dénomme pTG7904, dépourvu de la séquence codant pour le dipeptide Ala-Pro a été également construit par mutagénèse dirigée de M13TG7740 à l'aide de l'oligonucléotide OTG2299 et insertion d'un fragment de restriction SpHI dans pTG3828. La figure 2 résume le protocole d'obtention de pTG7908, pTG7904, et pTG7941.

### EXEMPLE 2 : Production du variant Ala-Pro-trophoblastine par la levure.

Une souche de levure de l'espèce *Saccharomyces cerevisiae* de génotype MATalpha, ura3-251,-373,-328, leu2-3,-112,his3,pep4-3 est transformée par le plasmide pTG7908 par la méthode à l'acétate de lithium [H. ITO, et al., J. Bacteriol. (1983) 153] et les prototrophes pour l'uracile (Ura$^+$) sont sélectionnées sur un milieu YNBG (14 g/l de bases azotées pour levure (Yeast Nitrogen Base), 10 g/l de glucose) additionné avec 10 g/l de casaminoacides.

Pour comparer le taux d'expression des différents variants codés par les plasmides décrits à l'exemple 1, des clones de cellules transformées par ces plasmides ont été cultivés en flacons à 30°C, jusqu'à une DO$_{600}$ de 8 à 10 unités, afin de déterminer la production de trophoblastine dans les surnageants de culture.

La production de trophoblastine recombinante par les levures transformées respectivement par les plasmides pTG 7904, pTG 7908 et pTG 7941 a été évaluée, par électrophorèse sur gel d'acrylamide, coloration au bleu de Coomassie et comparaison avec une préparation de protéine standard (PHARMACIA).

Les résultats sont illustrés par le tableau I suivant :

TABLEAU I

| Plasmide | Trophoblastine recombinante (mg/l/DO$_{600}$) |
|---|---|
| pTG 7904 | 0,06 à 0,25 |
| pTG 7908 | 2 à 2,5 |
| pTG 7941 | 1 à 1,5 |

Ces résultats montrent que la présence de l'extension N-terminale de 2 acides aminés entraîne une augmentation importante (de 4 à 10 fois) de la production de trophoblastine recombinante par la levure.

Le variant Ala-Pro-trophoblastine est produit par "Fed-Batch" dans un fermenteur de 20 L BIOLAFFITE. 12 l de milieu D käppeli [A. FICCHTER et al., Adv. Microbial. Physiol. (1981), 22, 123-183] concentré 1,5 fois, qui contiennent

# EP 0 513 336 B1

10 g/l de glucose et de HY Case SF (vendu par SHEFFIELD) sont ensemencés avec 400 ml d'une préculture d'un clone de levure transformé par pTG9708. Cette préculture est effectuée en erlenmeyer à 30°C sur un milieu sélectif YNBG. Avant la mise en route de la fermentation, la $DO_{600}$ du milieu ainsi ensemencé est de 0,2. La fermentation se fait à 30°C, à un pH de 4,5 contrôlé par adjonction d'une solution d'ammoniaque à 10% et à une pression partielle d'oxygène correspondant à 30% de la pression de saturation, maintenue constante par une régulation de l'agitation. Lorsque tout le glucose est consommé, la $DO_{600}$ est mesurée (DO de départ de l'alimentation) et on commence l'alimentation en glucose par paliers exponentiels de 3 heures, sachant que le taux de croissance spécifique (μ) est de 0,1 heure$^{-1}$ et que la quantité de glucose rajoutée (QS) est de 0,045 g/h/DO. La fermentation est arrêtée lorsque $DO_{600}$ = 100, et la récolte se fait par centrifugation à 5000g. On obtient ainsi 13 l de surnageant de culture contenant le variant Ala-Pro-trophoblastine en grande quanité.

**EXEMPLE 3 : Purification du variant Ala-Pro-trophoblastine à partir des surnageants de culture**

- A l'échelle semi-préparative

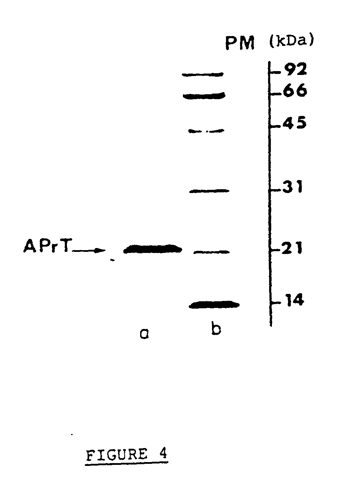

Les surnageants de culture de levures sont centrifugés puis concentrés et dialysés contre du tampon Tris-HCl, 0,05M, (pH 8,2) dans une cellule d'ultrafiltration (AMICON), sur membrane FILTRON retenant les molécules supérieures à 10 KDa. L'isolement de l'APrT a été effectué à l'échelle semi-préparative par chromatographie liquide à haute performance (HPLC), sur une colonne d'échangeur d'anions TSK DEAE-5PW semi-préparative (150 x 21,5 mm) équilibrée par du tampon Tris-HCl, 0,05 M, pH 8,2. Le débit est de 4 ml/min. Après injection de l'échantillon, on procède à l'élution par un gradient de KCl de 0 à 0,135 M (tampon Tris-HCl, 0,05 M, pH 8,2) pendant 90 min, suivi d'une phase de plateau isocratique à 0,135 M de KCl pendant 30 min, puis d'un second gradient, jusqu'à 0,5 M de KCl pendant 80 min. L'élution est suivie par mesure de l'absorption à 280 nm. Le pic correspondant à l'APrT est identifié à l'aide d'un immunsérum anti-trophoblastine naturelle (la similitude des propriétés immunologiques de l'APrT et de la trophoblastine naturelle est démontrée ci-dessous, à l'exemple 4), puis les fractions correspondantes sont recueillies. L'APrT sort à la fin de la phase de plateau isocratique entre 0,135 M et 0,25 M de KCl.

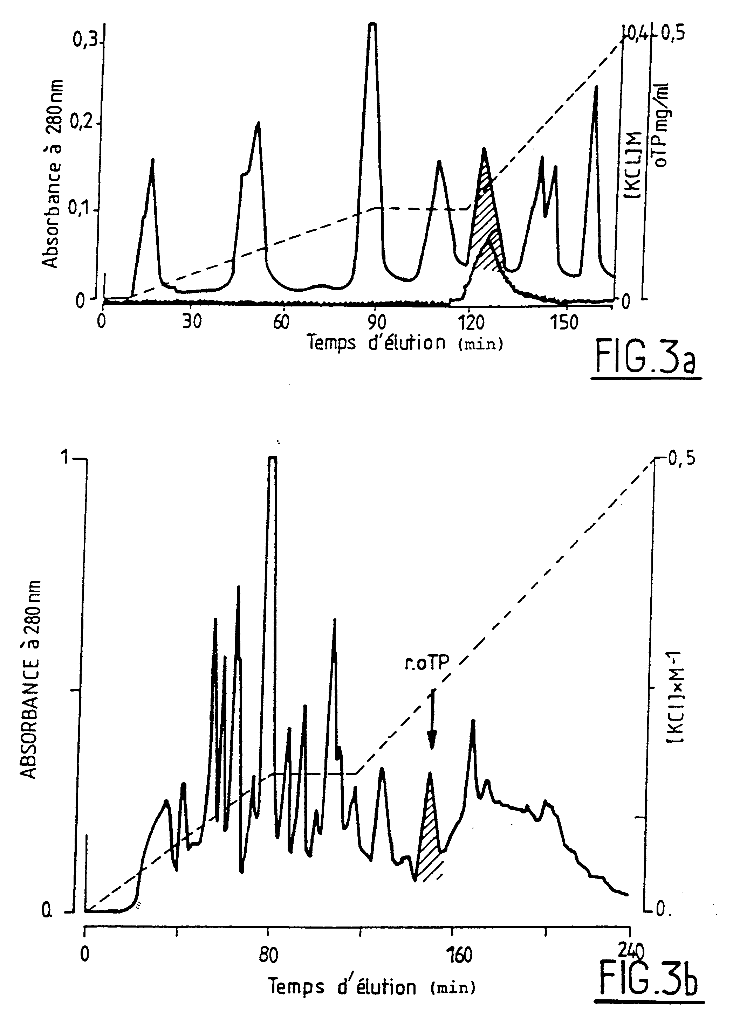

La figure 3(a) illustre le profil d'élution obtenu ; le pic correspondant à l'APrT est hachuré.
Le poids moléculaire apparent de l'APrT en électrophorèse sur gel de polyacrylamide en présence de SDS est d'environ 21 KDa. La figure 4 montre le profil électrophorétique obtenu (puits a : APrT ; puits b : marqueurs de poids moléculaire).

- Purification préparative

La purification est effectuée sur colonne TSK DEAE-5PW préparative (200 x 55 mm). L'élution est effectuée par un gradient de KCl dans un tampon Tris-HCl 0,05 M pH 8,3 à un débit de 25 ml/min, dans les conditions suivantes :

- gradient de 0 à 0,135 M KCl pendant 80 min ;
- phase isocratique à 0,135 M pendant 40 min ;
- gradient de 0,135 à 0,5 M KCl pendant 120 min.

Le profil d'élution est représenté à la figure 3(b). Le pic hachuré correspond à l'APrT.

**EXEMPLE 4 : Comparaison des propriétés immunologiques de l'APrT avec celles de la trophoblastine naturelle**

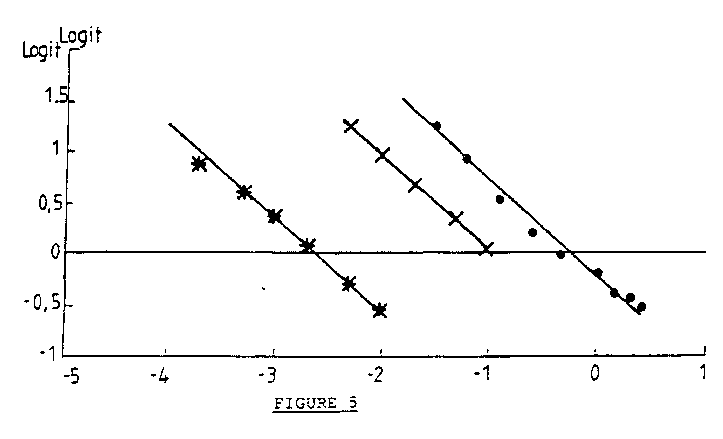

L'existence de réactions croisées immunologiques entre l'APrT et la trophoblastine est évaluée par radioimmunoessai (RIA), en utilisant un antisérum polyclonal anti-trophoblastine, et une préparation de trophoblastine marquée à l'iode 125, selon le protocole décrit dans la Demande PCT 89/08706.
Les courbes RIA d'inhibition obtenues dans ces conditions sont représentées à la figure 5 ; en abscisse est porté le Log de la dilution, et en ordonnée le Logit = Log (Bo/1-Bo) correspondant (Bo représente la concentration maximale de trophoblastine liée, pour une concentration constante d'antisérum anti-trophoblastine)

- (•) témoin : préparation purifiée de trophoblastine naturelle ;

$$Y= - 0,9572X - 0,2341$$

coefficient de régression linéaire = 0,9957
- (×) milieu de culture des levures sécrétant l'APrT (dilutions de 1 à 1/100)

$$Y = -0,9372X - 0,921$$

coefficient de régression linéaire = O,9996

- (∗) préparation d'APrT (dilutions de 1 à 1/4000) obtenue comme décrit à l'exemple 1,

$$Y = -0,9341X - 2,41$$

coefficient de régression linéaire = 0,9955

Ces courbes sont parallèles entre elles, ce qui démontre que les propriétés immunologiques de l'APrT sont similaires à celles de la trophoblastine naturelle.

## EXEMPLE 5 : Test d'activité antivirale

L'activité antivirale de l'APrT est mesurée selon le protocole décrit par LA BONNARDIERE et LAUDE, [Infection and Immunity, 32, 28-31, (1981)], sur des cellules MDBK (Madin Darby Bovine Kidney), une lignée de cellules de rein de veau, en présence du virus de la stomatite vésiculaire.

Les résultats obtenus sont comparés à ceux obtenus à partir d'un interféron de référence, qui est un interféron α porcin, d'un titre de 1 000 UI, lui-même étalonné par rapport au standard humain de référence .

Les résultats de ce test d'activité antivirale font apparaître une activité équivalente, pour les surnageants de milieu de culture de levures (0,8 x $10^8$ UI/mg), l'APrT purifiée (0,55 x $10^8$ UI/mg), et la trophoblastine naturelle (0,7 x $10^8$ UI/mg) (la quantité de trophoblastine est déterminée par dosage radioimmunologique).

## EXEMPLE 6 : Démonstration *in vivo* de l'activité antilutéolytique de l'APrT

### Animaux et traitement hormonal

L'expérimentation a été menée chez 30 brebis de race Préalpes-du-Sud. Les cycles oestriens sont synchronisés au moyen d'éponges vaginales imprégnées de 300 mg de 17α-acétoxy-9α-fluoro-11β-hydroxyprogestérone (SEARLE, INTERVET). Ces éponges sont laissées en place pendant 14 jours et le jour du retrait (J14), on injecte aux brebis, par voie intramusculaire, 500 UI de PMSG (pregnant mare sérum gonadotrophin) ; 48h après, les brebis commencent un nouveau cycle (JO).

### Mise en place de cathéters intra-utérins

Les brebis sont anesthésiées, puis, par laparotomie au niveau de la ligne blanche, l'opérateur accède à l'utérus et procède au marquage des corps jaunes à l'encre de Chine. Un cathéter stérile (SILASTIC, DOW CORNING) de 0,076 mm de diamètre interne, 0,165 mm de diamètre externe et de 70 cm de long est équipé d'un court manchon à l'une de ses extrémités, qui permet de maintenir en place le cathéter dans la corne utérine, sensiblement au niveau de la jonction utéro-tubaire. L'ouverture est refermée avec une aiguille sertie à un fil "catgut" (Laboratoire BRUNEAU). Une suture en bourse est effectuée autour de l'orifice d'insertion du cathéter assurant le positionnement durable du dispositif. Un point de sécurité réalisé avec une aiguille sertie à de la soie (Laboratoire BRUNEAU) fixe le cathéter au ligament large. L'autre extrémité est fermée avec un fil de lin et une boucle est pratiquée, afin que l'opérateur, après avoir percé la paroi abdominale au niveau du flanc droit, puisse ressortir le cathéter en tirant sur la boucle. Un frein de SILASTIC a été placé à 30 cm de l'extrémité intra-utérine pour limiter la sortie du cathéter et servir de butée à l'intérieur de la paroi abdominale. Une longueur d'environ 40 cm est à la disposition de l'opérateur qui procède aux injections intra-utérines. Un point effectué autour de l'orifice abdominale d'émergence du cathéter, renforce la solidité du dispositif. Les poses de cathéters sont pratiquées entre les 9e et 11e jours du cycle.

### Injections intra-utérines

Trois lots de brebis ont été constitués : les injections intra-utérines commencent entre le 10ème et le 12ème jour du cycle, et sont effectuées 2 fois par jour, pendant 8 jours. L'APrT est en solution dans du sérum physiologique contenant 50 000 UI/ml de pénicilline G et de la BSA (Sérumalbumine Bovine) à 2‰. Le volume de solution injecté est de 1ml.

. **Lot A :** C'est un lot témoin composé de 10 brebis qui reçoivent 2 fois par jour 1 ml de solution de BSA (fraction V, SIGMA) à 2‰ dans un sérum physiologique (NaCl 0,9%) contenant 50 000 UI/ml de pénicilline G.
. **Lot B :** Ce groupe est composé de 8 brebis auxquelles on a administré bi-quotidiennement 170 µg d'APrT.
. **Lot C :** Ce groupe est composé de 4 brebis qui reçoivent 2 fois par jour 80 µg d'APrT
. **Lot D :** 5 brebis reçoivent 2 fois par jour 340 µg d'APrT.

En fin d'expérimentation, toutes les brebis subissent une laparotomie exploratrice afin, d'une part, de vérifier que les cathéters sont restés bien en place et, d'autre part, de contrôler dans chaque lot la présence ou non de corps jaune(s) marqué(s) à l'encre de Chine.

## Dosage radioimmunologique de la progestérone

Le sang des animaux est prélevé dans la veine jugulaire à l'aide de tubes Vacutainer (BECTON-DICKINSON) sans anticoagulant. La concentration de progestérone sérique est déterminée par dosage radioimmunologique direct sans extraction, selon le protocole décrit par HEYMAN et al. [J.Reprod.Fert., 70, 533-540, (1984)]. De la progestérone marquée au tritium et un immunsérum anti-progestérone spécifique (INSTITUT PASTEUR) sont utilisés pour ce dosage.

Dans le lot A témoin, la concentration de progestérone dans le sang périphérique diminue brutalement chez l'ensemble des brebis à partir du 14è jour, pour atteindre des taux inférieurs à 0,5 ng/ml entre le 15e et le 17e jour post-oestrus. La durée moyenne du cycle est, dans ce lot, de 15,2 ± 0,3 jours. L'administration de 80 µg d'APrT par jour (lot C) ne prolonge pas cette durée.

Dans le lot B, (170 µg/jour) on constate au 14e jour du cycle un ralentissement de la chute de la progestéronémie par rapport au lot A : 7 brebis sur 8 présentent à ce stade des taux de progestéronémie supérieurs à 1 ng/ml (contre 4 sur 10 dans le lot A) ; au 15e jour du cycle, 5 brebis sur 8 présentent encore des taux de progestéronémie supérieurs à 1 ng/ml (contre 2 sur 10 dans le lot A).

Dans ce lot, la lutéolyse est retardée en moyenne de 2 jours par rapport au lot A.

Dans le lot D, l'administration intra-utérine d'APrT à la dose de 340 µg/jour maintient la fonction lutéale bien au-delà de la durée du cycle normal chez quatre brebis sur 5 (25, 32, 45, et 64 jours respectivement, chez les brebis n°9037. 9431, 9458, et 9053).

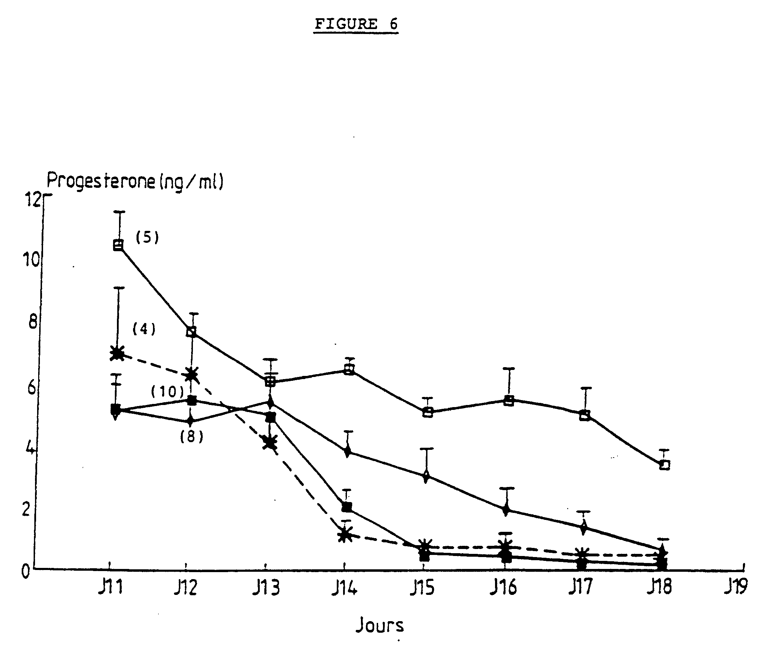

Le profil moyen comparatif de sécrétion de progestérone entre les différents lots, représenté par la Figure 6 montre bien qu'il existe une persistance nette de l'activité lutéale dans le lot D.

(■) Lot A
(◊) Lot B
(∗) Lot C
(□) Lot D

En outre, lors du contrôle chirurgical par laparotomie, aucun corps jaune néoformé n'a été constaté chez aucune des 4 brebis du lot D mentionnées ci-dessus, ce qui montre que la progestéronémie mesurée correspond à la persistance des corps jaunes cycliques antérieurs aux injections d'APrT.

D'éventuels effets secondaires de l'APrT ont été recherchés : la température moyenne des brebis des lots testés a été prise quotidiennement.

Aucune différence n'a été observée entre la température des animaux témoins du lot A et celle des animaux des autres lots.

De façon plus générale, aucune perturbation du comportement (perte d'appétit, etc...) n'a été observée chez les animaux traités par l'APrT, par rapport aux animaux témoins. Il en est de même de la formule sanguine (globules rouges, globules blancs, plaquettes) et des taux de transaminases sériques (SGPT).

## - Injections intra-musculaires

La même expérimentation a été effectuée, en injectant l'APrT par voie intra-musculaire.

Un lot de brebis (E) a reçu quotidiennement 2 injections de solution d'APrT (2 mg d'APrT/jour : 1 mg le matin, 1 mg le soir.

Un lot de brebis témoin (F) a reçu des injections d'une solution de SAB (2 mg/jour : 1 mg le matin, 1 mg le soir).

La persistance de l'activité lutéale chez les animaux du lot E est similaire à celle observée chez les animaux du lot D (traités par injection intra-utérine).

Le seul effet secondaire observé est une légère élévation de la température moyenne des animaux du lot E par rapport à ceux du lot F. En revanche, aucune modification du comportement n'est observée, ainsi que des formules sanguines et des taux de transaminases sériques (SGPT).

Des expériences précédemment réalisées montraient l'activité antilutéolytique de la trophoblastine naturelle, mais elles ne permettaient pas d'affirmer que la trophoblastine suffisait à elle seule pour prévenir la lutéolyse, et n'avaient pas permis de déterminer quel était le rôle des différentes isoformes de la trophoblastine. Or, les expériences décrites ci-dessus montrent que l'APrT obtenue à partir d'une seule isoforme suffit, à des doses appropriées, à inhiber la lutéolyse, et ce, en dépit des deux aminoacides supplémentaires de l'extrémité N-terminale. Enfin, aucun apparent signe de toxicité de proline n'est observé.

## EXEMPLE 7 : Démonstration des propriétés immunosuppressives de l'APrT

Ces propriétés ont été démontrées par quatre typés de tests permettant la mise en évidence de modes d'action différents

- l'activité antimitotique, évaluée par l'action sur la prolifération des lymphocytes murins, humains ou ovins ;
- l'activité d'inhibition de la réaction cytolytique de rejet de greffe, évaluée grâce au test *in vitro* de la réaction lymphocytaire mixte (RLM);
- l'activité d'inhibition de la réaction locale de rejet de greffe *in-vivo* (Local Graft versus Host reaction).
- l'activité immunorégulatrice sur la population des lymphocytes tueurs NK, indépendants des antigènes du complexe majeur d'histocompatibilité (MHC).

### 1) Action de l'APrT sur la prolifération des lymphocytes murins activés par la phytohémagglutinine

Des lymphocytes murins sont obtenus à partir de rate de souris C3H/He ou Balb/c après broyage au Potter et lavage deux fois dans du milieu de culture RPMI 1640 à 1500 t/mn pendant 10 mn. Enfin, les lymphocytes isolés sont mélangés dans le même milieu de culture, additionné de 10 % de sérum de veau foetal (SVF), à une concentration finale de $5.10^6$ cellules/ml. Le milieu de culture est composé de 500 ml de RPMI 1640 (GIBCO) + 5 ml de pénicilline G/streptomycine (GIBCO) + 5 ml de bicarbonate de sodium 7,5 % (GIBCO) + 5 ml de glutamine.

100 µl par puits de milieu de culture contenant $6 \times 10^5$ lymphocytes, activés par 5 µg/ml de phytohémagglutinine (PHA) (WELLCOME) sont incubés avec 100 µl d'une solution d'APrT à 3 µg/ml ($\approx 10^8$ UI/mg) ou 100 µl de milieu de culture (contrôle) dans des plaques microtest de 96 puits à 37°C, dans une atmosphère d'air-$CO_2$ (95 %-5 %), pendant 48h.

La prolifération lymphocytaire est évaluée par mesure de l'incorporation de thymidine tritiée. 25 µl de thymidine $^3H$ (0,04 mCi/ml) sont ajoutés à chaque puits et les cellules sont récoltées, 24 h après et déposées sur filtre (Glass Microfibre Filters-GFM-WHATMAN). Après séchage, les filtres sont déposés dans des tubes auxquels on ajoute 1 ml de liquide de scintillation (ECONOFLUOR). La radioactivité est mesurée dans un compteur à rayonnement β (BECKMAN).

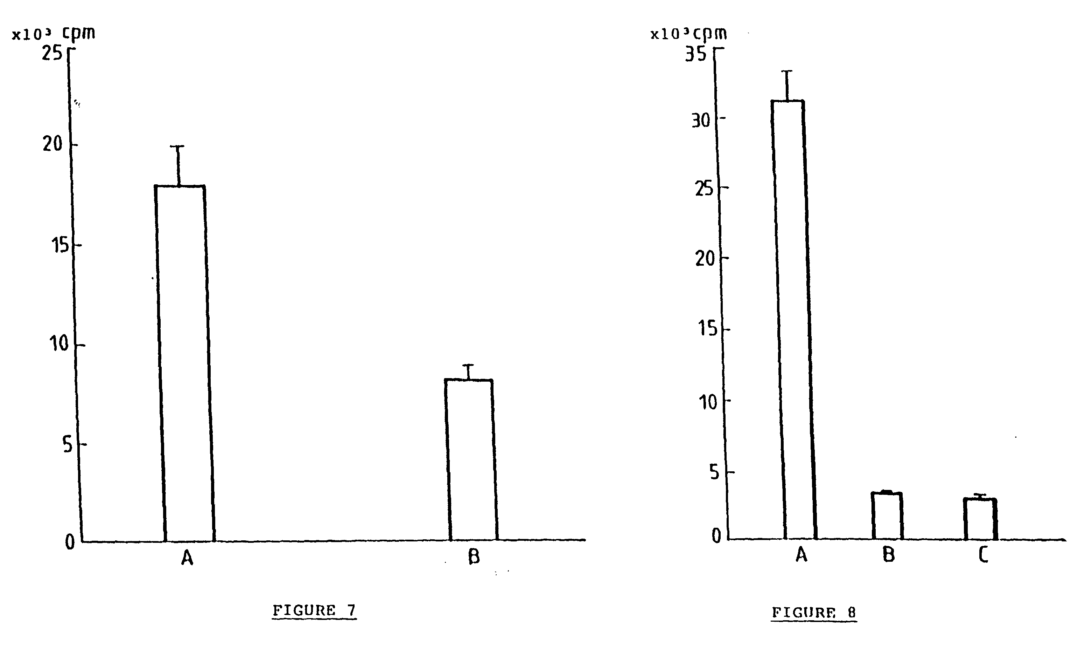

Les résultats sont illustrés par la Figure 7, qui montre que l'APrT inhibe de façon très marquée (55 %) la prolifération des lymphocytes murins traités à la PHA.

A : Témoin
B : APrT.

La radioactivité en c.p.m. est portée en ordonnée. En présence de lymphocytes humains ou ovins activés par la phytohémagglutinine A (PHA), l'APrT inhibe également la réplication lymphocytaire. Cette inhibition ne résulte pas d'un effet cytotoxique de l'APrT, car la viabilité cellulaire n'est pas affectée par l'APrT, ce qui est montré par incubation des lymphocytes en présence de bleu Trypan ou de $^{51}Cr$.

### 2) Action de l'APrT sur une réaction lymphocytaire mixte

Les réactions lymphocytaires mixtes sont réalisées en incubant par puits 150 µl de milieu de culture contenant $5.10^8$ cellules réactives C3H/He par ml avec $5.10^6$ cellules stimulatrices, isogéniques ou allogéniques, irradiées à 1 800 Rads/ml.

On utilise des cellules de souris C3H/He pour la réaction isogénique et des cellules de souris Balb/c pour la réaction allogénique. On ajoute par puits 100 µl d'APrT à 3 µg/ml ($10^8$ UI/mg) ou de milieu de culture (contrôle) dans des plaques microtest de 96 puits (FALCON 3072) à 37 °C, et on laisse les cultures sous atmosphère gazeuse d'air-$CO_2$ (95 % - 5 %) pendant 4 jours.

La lyse lymphocytaire par les lymphocytes cytotoxiques produits au cours de la réaction lymphocytaire mixte est évaluée en mesurant l'incorporation de thymidine tritiée. 25 µl de thymidine $^3H$ sont ajoutés 24 h avant le prélève-

**EP 0 513 336 B1**

ment des lymphocytes et leur dépôt sur filtre. La mesure de la radioactivité des filtres est effectuée dans un compteur à scintillation.

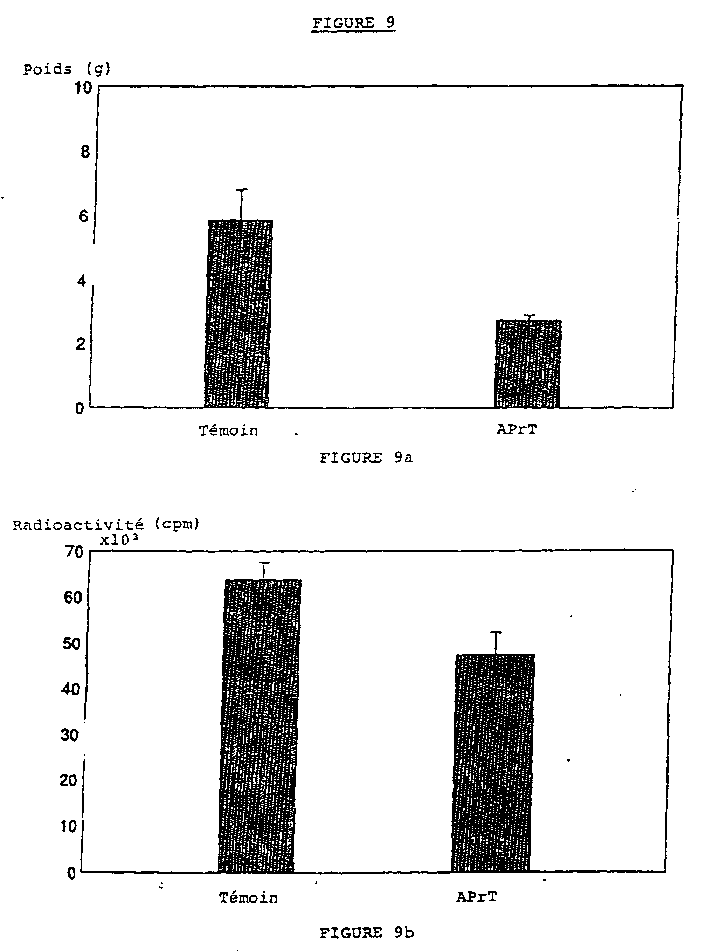

Les résultats sont représentés Figure 8 :

A : Contrôle
B : Trophoblastine naturelle
C : APrT

La radioactivité en c.p.m. est portée en ordonnée.

En culture bidirectionnelle de lymphocytes provenant de deux souches de Souris (Balb/c et C3H/He), l'APrT inhibe à 90% la lyse des lymphocytes murins par les cellules cytotoxiques (CTL) produites.

**3) Réaction locale de rejet de greffe**

L'APrT à raison de 2 μg/ml, est ajoutée à une suspension de cellules spléniques allogéniques provenant de souris Balb/c qui est injectée dans les coussinets plantaires d'une patte postérieure de souris receveuses $F_1$ (Balb/b x $B_6$).

L'autre patte postérieure, utilisée comme témoin, est injectée avec les cellules spléniques, mais sans APrT. Les ganglions lymphocytaires poplités sont prélevés 4 à 6 jours plus tard (selon les lots de souris) et pesés. Des cellules de ces ganglions sont prélevées et activées par la PHA ; l'incorporation de $^3$H thymidine dans ces cellules est mesurée.

Les résultats sont représentés sur les figures 9(a) et 9(b) qui montrent que le poids des ganglions lymphatiques dans les cellules traitées par l'APrT est diminué par rapport aux cellules témoins, et que l'incorporation de thymidine tritiée dans les cellules issues des ganglions poplités des pattes traitées par l'APrT est également moindre que dans les cellules témoins.

Ces résultats montrent que l'APrT inhibe *in-vivo* la réaction de rejet de greffe, et ce, même chez une espèce (souris) très éloignée de l'espèce ovine.

**4) Action de l'APrT sur la lyse cellulaire par les cellules NK**

Des cellules K562 (lignée érythroleucémique humaine) sont centrifugées 10 mn à 1800 t/mn et 0,5 ml de $^{51}$Cr sont déposés sur le culot. Après 1 h d'incubation à 37 °C dans 5 % de $CO_2$-95 % d'air, les cellules sont lavées trois fois dans du milieu de culture RPMI et resuspendues dans le même milieu de culture, à la concentration de 2 X $10^5$ cellules/ml.

Trois types d'incubation en plaque de microtitration à 37 °C, dans une atmosphère air-$CO_2$ (95 % - 5 %) sont réalisées en parallèle:

- 100 μl de cellules K562 marquées + 100 μl de lymphocytes humains 50 à 100 fois plus concentrés + 100 μl d'APrT à 3 μg/ml ($10^8$ UI/mg) ou de milieu de culture (témoin) permettent de déterminer les protéines radioactives du milieu expérimental (prot. rad. exp.) ;
- 100 μl de cellules K562 marquées + 200 μl d'HCl 4N permettent d'évaluer les protéines radioactives du milieu total (prot. rad. tot.) ;
- 100 μl de K562 marquées + 200 μl de milieu de culture permettent de déterminer les protéines radioactives du milieu spontané (prot. rad. spont.).

Après 4 h d'incubation, 100 μl de surnageant sont prélevés dans chaque puits et la radioactivité due à la libération de protéines marquées au $^{51}$Cr dans le milieu est comptée en cpm dans un compteur à rayonnement gamma.

Les résultats sont exprimés en moyenne de pourcentage de lyse cellulaire d'échantillons et calculés selon la formule suivante :

$$\% \text{ de lyse} = \frac{\text{cpm prot. rad. exp.} - \text{cpm prot. rad. spont.}}{\text{cpm prot. rad. tot.} - \text{cpm prot. rad. spont.}}$$

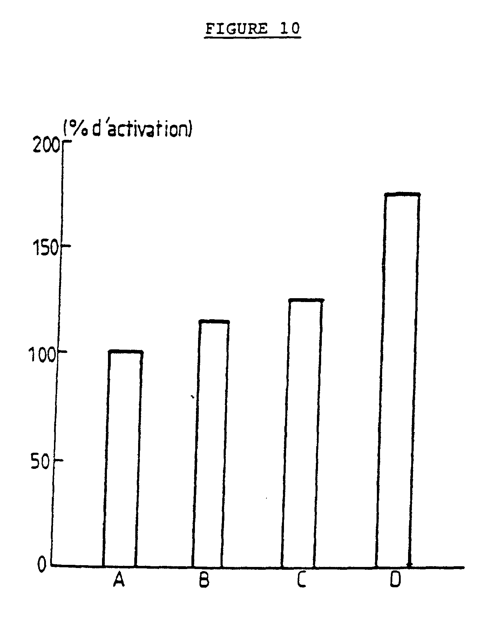

La figure 10 montre les résultats obtenus :

A : Contrôle
B : APrT
C : IFN alpha

D : IFN gamma

Il apparaît donc nettement que l'APrT active la lyse cellulaire des cellules cible K562 par les cellules NK.

Cette activation de la lyse cellulaire par les cellules NK est analogue à celle observée à partir d'interféron $\alpha$ humain de classe 1 et inférieure à celle de l'interféron $\gamma$ humain de référence.

Ainsi que cela ressort de ce qui précède, l'Invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente Invention.

**Revendications**

**Revendications pour les Etats contractants suivants : AT, BE, CH, DE, DK, FR, GB, IT, LI, LU, NL, SE**

**1.** Variant d'interféron, répondant à l'une des formules suivantes :

$$X_1\text{-}R_0 \tag{I}$$

ou bien

$$X_1\text{-}X_2\text{-}R_0 \tag{II}$$

ou bien

$$X_1\text{-}X_2\text{-}X_3\text{-}R_o \tag{III}$$

dans laquelle $X_1$, $X_2$ et $X_3$ sont identiques ou différents, et représentent chacun un acide aminé,
et $R_o$ représente la séquence en acides aminés de la forme mature d'un interféron de classe trophoblastine.

**2.** Variant selon la revendication 1, **caractérisé en ce que** $X_1$, $X_2$, et $X_3$ représentent chacun un acide aminé acide, un acide aminé basique, ou un acide aminé choisi dans le groupe constitué par l'alanine, la valine, la proline, la glycine, la sérine, la thréonine, la cystéine, l'asparagine, la glutamine, à condition que $X_1$ représente un acide aminé différent de la proline.

**3.** Variant selon l'une quelconque des revendications 1 ou 2, de formule :

$$\text{Ala-Pro-}R_0{'} \tag{IV}$$

ou

$$\text{Ala-Gly-}R_0{'} \tag{V}$$

dans laquelle $R_0{'}$ représente la séquence en acides aminés de la forme mature d'un IFN de classe trophoblastine.

**4.** Variant selon la revendication 3, de formule :

$$\text{Ala-Pro-}R_0{''} \tag{VI}$$

ou

$$\text{Ala-Gly-}R_0'' \qquad\qquad (VII)$$

dans laquelle $R_0''$ représente la séquence en acides aminés de la forme mature d'une trophoblastine d'origine bovine ou ovine.

**5.** Variant selon la revendication 4, de formule :

$$\text{Ala-Pro-}R_0''' \qquad\qquad (VIII)$$

ou

$$\text{Ala-Gly-}R_0''' \qquad\qquad (IX)$$

dans laquelle $R_0'''$ représente la forme mature de l'une quelconque des isoformes de la trophoblastine ovine.

**6.** Variant selon la revendication 5, dans lequel $R_0'''$ représente une séquence générale d'acide aminés des isoformes de la trophoblastine ovine commençant avec le résidu cystéine en position 1 et finissant avec le résidu proline en position 172, dans laquelle :

- . $R_5$ est un résidu acide glutamique, glutamine ou arginine,
- . $R_6$ est un résidu arginine ou lysine,
- . $R_{35}$ est un résidu lysine ou acide aspartique, et
- . $R_{44}$ est un résidu acide glutamique ou acide aspartique.
- . $R_{48}$ est un résidu leucine ou acide aspartique.
- . $R_{49}$ est un résidu leucine ou glutamine.

**7.** Variant selon la Revendication 6, sélectionné parmi :

- un variant de formule (Xa) dans laquelle $R_5$ est un résidu arginine, $R_6$ est un résidu lysine, $R_{35}$ est un résidu acide aspartique, $R_{44}$ est un résidu acide glutamique, $R_{48}$ est un résidu leucine, et $R_{49}$ un résidu glutamine,
- un variant de formule (Xb) dans laquelle $R_5$ est un résidu acide glutamique, $R_6$ est un résidu arginine, $R_{35}$ est un résidu lysine, $R_{44}$ est un résidu acide glutamique, $R_{48}$ est un résidu acide aspartique, et $R_{49}$ un résidu leucine,.
- un variant de formule (Xc) dans laquelle $R_5$ est un résidu glutamine, $R_6$ est un résidu arginine, $R_{35}$ est un résidu acide aspartique, et $R_{44}$ est un résidu acide aspartique, et,
- un variant de formule (Xd) laquelle $R_5$ est un résidu glutamine, $R_6$ est un résidu arginine, $R_{35}$ est un résidu acide aspartique, $R_{44}$ est un résidu acide glutamique, $R_{48}$ est un résidu leucine, et $R_{49}$ un résidu glutamine.

**8.** Cassette d'expression d'un variant selon l'une quelconque des revendications 1 à 7, qui comprend au moins :

- un premier fragment d'ADN codant pour ledit variant,
- un deuxième fragment d'ADN codant pour un peptide signal, ledit deuxième fragment d'ADN étant associé à l'extrémité 5' du premier fragment d'ADN,
- un promoteur permettant l'expression desdits fragments d'ADN dans la levure.

**9.** Cassette d'expression selon la revendication 8, dans laquelle le deuxième fragment d'ADN code pour le peptide signal du précurseur du facteur-$\alpha$, pour le peptide signal du précurseur de la $\beta$-1,-3 glucanase de levure ou un dérivé fonctionnel de ceux-ci.

**10.** Cassette d'expression selon une quelconque des revendications 8 ou 9, dans laquelle le promoteur est celui du gène MF$\alpha$1 ou un dérivé fonctionnel de celui-ci.

**11.** Cellule de levure transformée par une cassette d'expression selon n'importe laquelle des revendications 8 à 10.

**12.** Procédé de production d'un variant selon l'une quelconque des revendications 1 à 7, qui comprend l'acte de cultiver une cellule de levure selon la revendication 11, et de récolter ledit variant à partir du surnageant de culture.

**13.** Procédé selon la Revendication 12, **caractérisé en ce que** ledit variant est purifié à partir du surnageant de culture, par une chromatographie d'échange d'anions comprenant :

- une élution par un gradient de KCl de O à 0,135 M

- une phase isocratique à 0,135 M de KCl

- une élution par un gradient de KCl de 0,135 M à 0,5 M,

   ledit variant étant recueilli entre 0,135 M et 0,3 M de KCl.

**14.** Utilisation d'un variant d'interféron selon l'une quelconque des Revendications 1 à 7, pour l'obtention d'un médicament.

**15.** Utilisation d'un variant d'interféron selon l'une quelconque des Revendications 1 à 7, pour l'obtention d'un médicament destiné à une utilisation thérapeutique choisie parmi :

- une utilisation antiinflammatoire ;
- une utilisation antivirale ;
- une utilisation antitumorale ;
- une utilisation immunomodulatrice ;
- une utilisation antilutéolytique.

**16.** Utilisation d'un variant d'interféron selon l'une quelconque des Revendications 1 à 7, pour l'obtention d'une composition immunogène.

**17.** Utilisation d'un variant d'interféron selon l'une quelconque des Revendications 1 à 7, pour l'obtention d'une composition destinée au traitement des embryons lors de leur transplantation.

**18.** Utilisation d'un variant d'interféron selon l'une quelconque des Revendications 1 à 7, pour l'obtention d'un réactif de diagnostic.

**19.** Utilisation d'un variant d'interféron selon l'une quelconque des Revendications 1 à 7, pour l'obtention d'un réactif destiné au diagnostic de viabilité des embryons à un stade précoce de leur développement.

**Revendications pour les Etats contractants suivants : ES, GR**

**1.** Procédé de préparation d'un variant d'interféron, **caractérisé en ce que** l'on prépare un variant répondant à l'une des formules suivantes :

$$X_1\text{-}R_0 \tag{I}$$

ou bien

$$X_1\text{-}X_2\text{-}R_0 \tag{II}$$

ou bien

$$X_1\text{-}X_2\text{-}X_3\text{-}R_o \qquad\qquad (III)$$

dans laquelle $X_1$, $X_2$ et $X_3$ sont identiques ou différents, et représentent chacun un acide aminé, et $R_o$ représente la séquence en acides aminés de la forme mature d'un interféron de classe trophoblastine.

2. Procédé selon la revendication 1, **caractérisé en ce que** l'on prépare un variant d'interféron dans lequel $X_1$, $X_2$, et $X_3$ représentent chacun un acide aminé acide, un acide aminé basique, ou un acide aminé choisi dans le groupe constitué par l'alanine, la valine, la proline, la glycine, la sérine, la thréonine, la cystéine, l'asparagine, la glutamine, à condition que $X_1$ représente un acide aminé différent de la proline.

3. Procédé selon une quelconque des revendications 1 ou 2, **caractérisé en ce que** l'on prépare un variant d'interféron de formule :

$$\text{Ala-Pro-}R_0'\cdot \qquad\qquad (IV)$$

ou

$$\text{Ala-Gly-}R_0' \qquad\qquad (V)$$

dans laquelle $R_0'$ représente la séquence en acides aminés de la forme mature d'un IFN de classe trophoblastine.

4. Procédé selon la revendication 3, **caractérisé en ce que** l'on prépare un variant d'interféron de formule :

$$\text{Ala-Pro-}R_0'' \qquad\qquad (VI)$$

ou

$$\text{Ala-Gly-}R_0'' \qquad\qquad (VII)$$

dans laquelle $R_0''$ représente la séquence en acides aminés de la forme mature d'une trophoblastine d'origine bovine ou ovine.

5. Procédé selon la revendication 4, **caractérisé en ce que** l'on prépare un variant d'interféron de formule :

$$\text{Ala-Pro-}R_0''' \qquad\qquad (VIII)$$

ou

$$\text{Ala-Gly-}R_0''' \qquad\qquad (IX)$$

dans laquelle $R_0'''$ représente la forme mature de l'une quelconque des isoformes de la trophoblastine ovine.

6. Procédé selon la revendication 5, **caractérisé en ce que** l'on prépare un variant d'interféron dans lequel $R_0'''$ représente une séquence générale d'acide aminés des isoformes de la trophoblastine ovine commençant avec le résidu cystéine en position 1 et finissant avec le résidu proline en position 172, dans laquelle :

. $R_5$ est un résidu acide glutamique, glutamine ou arginine,
. $R_6$ est un résidu arginine ou lysine,
. $R_{35}$ est un résidu lysine ou acide aspartique, et

- $R_{44}$ est un résidu acide glutamique ou acide aspartique.
- $R_{48}$ est un résidu leucine ou acide aspartique.
- $R_{49}$ est un résidu leucine ou glutamine.

7. Procédé selon la revendication 6, **caractérisé en ce que** l'on prépare un variant d'interféron sélectionné parmi :

- un variant de formule (Xa) dans laquelle $R_5$ est un résidu arginine, $R_6$ est un résidu lysine, $R_{35}$ est un résidu acide aspartique, $R_{44}$ est un résidu acide glutamique, $R_{48}$ est un résidu leucine, et $R_{49}$ un résidu glutamine,

- un variant de formule (Xb) dans laquelle $R_5$ est un résidu acide glutamique, $R_6$ est un résidu arginine, $R_{35}$ est un résidu lysine, $R_{44}$ est un résidu acide glutamique, $R_{48}$ est un résidu acide aspartique, et $R_{49}$ un résidu leucine,

- un variant de formule (Xc) dans laquelle $R_5$ est un résidu glutamine, $R_6$ est un résidu arginine, $R_{35}$ est un résidu acide aspartique, et $R_{44}$ est un résidu acide aspartique, et,

- un variant de formule (Xd) laquelle $R_5$ est un résidu glutamine, $R_6$ est un résidu arginine, $R_{35}$ est un résidu acide aspartique, $R_{44}$ est un résidu acide glutamique, $R_{48}$ est un résidu leucine, et $R_{49}$ un résidu glutamine.

8. Procédé de préparation d'une cassette d'expression d'un variant d'interféron tel que défini dans l'une quelconque des revendications 1 à 7, **caractérisé en ce que** l'on prépare une cassette d'expression qui comprend au moins :

- un premier fragment d'ADN codant pour ledit variant,

- un deuxième fragment d'ADN codant pour un peptide signal, ledit deuxième fragment d'ADN étant associé à l'extrémité 5' du premier fragment d'ADN,

- un promoteur permettant l'expression desdits fragments d'ADN dans la levure.

9. Procédé selon la revendication 8, cractérisé en ce que l'on prépare une cassette d'expression dans laquelle le deuxième fragment d'ADN code pour le peptide signal du précurseur du facteur-$\alpha$, pour le peptide signal du précurseur de la $\beta$-1,-3 glucanase de levure ou un dérivé fonctionnel de ceux-ci.

10. Procédé selon une quelconque des revendications 8 ou 9, **caractérisé en ce que** l'on prépare une cassette d'expression dans laquelle le promoteur est celui du gène MF$\alpha$1 ou un dérivé fonctionnel de celui-ci.

11. Procédé de préparation d'une cellule de levure transformée, **caractérisé en ce que** l'on transforme ladite cellule de levure par une cassette d'expression selon n'importe laquelle des revendications 8 à 10.

12. Procédé de production d'un variant d'interféron selon l'une quelconque des revendications 1 à 7, qui comprend l'acte de cultiver une cellule de levure transformée obtenue par un procédé selon la revendication 11, et de récolter ledit variant à partir du surnageant de culture.

13. Procédé selon la Revendication 12, **caractérisé en ce que** ledit variant est purifié à partir du surnageant de culture, par une chromatographie d'échange d'anions comprenant :

- une élution par un gradient de KCl de 0 à 0,135 M
- une phase isocratique à 0,135 M de KCl
- une élution par un gradient de KCl de 0,135 M à 0,5 M,

ledit variant étant recueilli entre 0,135 M et 0,3 M de KCl.

14. Procédé de préparation d'un médicament, **caractérisé en ce que** l'on utilise un variant d'interféron selon l'une quelconque des Revendications 1 à 7.

15. Procédé de préparation d'un médicament, destiné à une utilisation thérapeutique choisie parmi :

- une utilisation antiinflammatoire ;

- une utilisation antivirale ;
- une utilisation antitumorale ;
- une utilisation immunomodulatrice ;
- une utilisation antilutéolytique ;

**caractérisé en ce que** l'on utilise un variant d'interféron selon l'une quelconque des Revendications 1 à 7.

16. Procédé de préparation d'une composition immunogène, **caractérisé en ce que** l'on utilise un variant d'interféron selon l'une quelconque des Revendications 1 à 7.

17. Procédé de préparation d'une composition destinée au traitement des embryons lors de leur transplantation, **caractérisé en ce que** l'on utilise un variant d'interféron selon l'une quelconque des Revendications 1 à 7.

18. Procédé de préparation d'un réactif de diagnostic, **caractérisé en ce que** l'on utilise un variant d'interféron selon l'une quelconque des Revendications 1 à 7.

19. Procédé de préparation d'un réactif destiné au diagnostic de viabilité des embryons à un stade précoce de leur développement, **caractérisé en ce que** l'on utilise un variant d'interféron selon l'une quelconque des Revendications 1 à 7.

**Patentansprüche**

**Patentansprüche für folgende Vertragsstaaten : AT, BE, CH, DE, DK, FR, GB, IT, LI, LU, NL, SE**

1. Interferonvariante entsprechend einer der folgenden Formeln:

$$X_1\text{-}R_0 \tag{I}$$

oder

$$X_1\text{-}X_2\text{-}R_0 \tag{II}$$

oder

$$X_1\text{-}X_2\text{-}X_3\text{-}R_0 \tag{III}$$

worin $X_1$, $X_2$ und $X_3$ gleich oder verschieden sind und jeweils für eine Aminosäure stehen, und $R_0$ für die Aminosäuresequenz der reifen Form eines Interferons der Trophoblastinklasse steht.

2. Variante nach Anspruch 1, **dadurch gekennzeichnet, dass** $X_1$, $X_2$ und $X_3$ jeweils für eine saure Aminosäure, eine basische Aminosäure oder eine Aminosäure, ausgewählt aus der Gruppe bestehend aus Alanin, Valin, Prolin, Glycin, Serin, Threonin, Cystein, Asparagin, Glutamin, stehen, mit der Maßgabe, dass $X_1$ für eine andere Aminosäure als Prolin steht.

3. Variante nach Anspruch 1 oder Anspruch 2 der Formel:

$$\text{Ala-Pro-}R_0{}' \tag{IV}$$

oder

$$\text{Ala-Gly-}R_0'' \qquad\qquad (V)$$

worin $R_0'$ für die Aminosäuresequenz der reifen Form eines IFN der Trophoblastinklasse steht.

4.  Variante nach Anspruch 3 der Formel:

$$\text{Ala-Pro-}R_0'' \qquad\qquad (VI)$$

oder

$$\text{Ala-Gly-}R_0'' \qquad\qquad (VII)$$

worin $R_0''$ für die Aminosäuresequenz der reifen Form eines Trophoblastins vom Rind oder Schaf steht.

5.  Variante nach Anspruch 4 der Formel

$$\text{Ala-Pro-}R_0''' \qquad\qquad (VIII)$$

oder

$$\text{Ala-Gly-}R_0''' \qquad\qquad (IX)$$

worin $R_0'''$ für die reife Form einer der Isoformen des Schaftrophoblastins steht.

6.  Variante nach Anspruch 5, worin $R_0'''$ für eine allgemeine Aminosäuresequenz der Isoformen des Schaftrophoblastins steht, die mit dem Cysteinrest in der Position 1 beginnt und mit dem Prolinrest in der Position 172 endet, worin:

-   $R_5$ ein Glutaminsäure-, Glutamin- oder Arginin-Rest ist,

-   $R_6$ ein Arginin- oder Lysin-Rest ist,

-   $R_{35}$ ein Lysin- oder Asparaginsäure-Rest ist, und

-   $R_{44}$ ein Glutaminsäure- oder Asparaginsäure-Rest ist,

-   $R_{48}$ ein Leucin- oder Asparaginsäure-Rest ist,

-   $R_{49}$ ein Leucin- oder Glutamin-Rest ist.

7.  Variante nach Anspruch 6, ausgewählt aus:

-   einer Variante der Formel (Xa) , worin $R_5$ ein Arginin-Rest ist, $R_6$ ein Lysin-Rest ist, $R_{35}$ ein Asparaginsäure-Rest ist, $R_{44}$ ein Glutaminsäure-Rest ist, $R_{48}$ ein Leucin-Rest ist und $R_{49}$ ein Glutamin-Rest ist,

-   einer Variante der Formel (Xb), worin $R_5$ ein Glutaminsäure-Rest ist, $R_6$ ein Arginin-Rest ist, $R_{35}$ ein Lysin-Rest ist, $R_{44}$ ein Glutaminsäure-Rest ist, $R_{48}$ ein Asparaginsäure-Rest. ist und $R_{49}$ ein Leucin-Rest ist,

-   einer Variante der Formel (Xc), worin $R_5$ ein Glutamin-Rest ist, $R_6$ ein Arginin-Rest ist, $R_{35}$ ein Asparaginsäure-Rest ist und $R_{44}$ ein Asparaginsäure-Rest ist, und

-   einer Variante der Formel (Xd) , worin $R_5$ ein Glutamin-Rest ist, $R_6$ ein Arginin-Rest ist, $R_{35}$ ein Asparaginsäure-

Rest ist, $R_{44}$ ein Glutaminsäure-Rest ist, $R_{48}$ ein Leucin-Rest ist und $R_{49}$ ein Glutamin-Rest ist.

8. Expressionskassette einer Variante nach einem der Ansprüche 1 bis 7, die mindestens umfasst:

   - ein erstes DNA-Fragment, das für die Variante codiert,

   - ein zweites DNA-Fragment, das für ein Peptidsignal codiert, wobei das zweite DNA-Fragment mit dem 5'-Ende des ersten DNA-Fragments verbunden ist,

   - einen Promotor, der die Expression der DNA-Fragmente in der Hefe gestattet.

9. Expressionskassette nach Anspruch 8, worin das zweite DNA-Fragment für das Peptidsignal des Vorläufers des Faktors $\alpha$ oder für das Peptidsignal des Vorläufers der $\beta$-1,-3- Glucanase der Hefe oder für ein funktionelles Derivat davon codiert.

10. Expressionskassette nach Anspruch 8 oder Anspruch 9, worin der Promotor des Gens MF$\alpha$1 oder ein funktionelles Derivat davon ist.

11. Hefezelle, die durch eine Expressionskassette nach einem der Ansprüche 8 bis 10 transformiert ist.

12. Verfahren zur Herstellung einer Variante nach einem der Ansprüche 1 bis 7, das die Stufe des Züchtens einer Hefezelle nach Anspruch 11 und des Erntens der Variante aus der Überstehenden Kulturlösung umfasst.

13. Verfahren nach Anspruch 12, **dadurch gekennzeichnet, dass** die Variante ausgehend von der Überstehenden Kulturlösung gereinigt wird durch eine Anionenaustauschchromatographie umfassend:

    - eine Eluierung durch einen KCL-Gradienten von 0 bis 0,135 M,

    - eine isokratische Phase von 0,135 M KCL,

    - eine Eluierung durch einen KCL-Gradienten von 0,135 M bis 0,5 M, wobei die Variante zwischen 0,135 M und 0,3 M KCL gesammelt wird.

14. Verwendung einer Interferonvariante nach einem der Ansprüche 1 bis 7, um ein Medikament zu erhalten.

15. Verwendung einer Interferonvariante nach einem der Ansprüche 1 bis 7, um ein Medikament zu erhalten, das für eine therapeutische Verwendung bestimmt ist, ausgewählt aus:

    - einer entzündungshemmenden Verwendung;

    - einer antiviralen Verwendung;

    - einer antitumoralen Verwendung;

    - einer Immunmodulierenden Verwendung;

    - einer antiluteolytischen Verwendung.

16. Verwendung einer Interferonvariante nach einem der Ansprüche 1 bis 7 zum Erhalt einer immunogenen Zusammensetzung.

17. Verwendung einer Interferonvariante nach einem der Ansprüche 1 bis 7 zum Erhalt einer Zusammensetzung, die zur Behandlung von Embryonen bei deren Transplantation bestimmt ist.

18. Verwendung einer Interferonvariante nach einem der Ansprüche 1 bis 7 zum Erhalt eines diagnostischen Reagenzes.

19. Verwendung einer Interferonvariante nach einem der Ansprüche 1 bis 7 zum Erhalt eines Reagenzes, das zur Diagnose der Lebensfähigkeit von Embryonen in einem frühen Stadium ihrer Entwicklung bestimmt ist.

**Patentansprüche für folgende Vertragsstaaten : ES, GR**

1.  Verfahren zur Herstellung einer Interferonvariante, **dadurch gekennzeichnet, dass** man eine Variante herstellt, die einer der folgenden Formeln entspricht:

$$X_1-R_0 \qquad\qquad (I)$$

oder

$$X_1-X_2-R_0 \qquad\qquad (II)$$

oder

$$X_1-X_2-X_3-R_0 \qquad\qquad (III)$$

worin $X_1$, $X_2$ und $X_3$ gleich oder verschieden sind und jeweils für eine Aminosäure stehen, und $R_0$ für die Aminosäuresequenz der reifen Form eines Interferons der Trophoblastinklasse steht.

2.  Verfahren nach Anspruch 1, **dadurch gekennzeichnet, dass** man eine Interferonvariante herstellt, worin $X_1$, $X_2$ und $X_3$ jeweils für eine saure Aminosäure, eine basische Aminosäure oder eine Aminosäure, ausgewählt aus der Gruppe bestehend aus Alanin, Valin, Prolin, Glycin, Serin, Threonin, Cystein, Asparagin, Glutamin, stehen, mit der Massgabe, dass $X_1$ für eine andere Aminosäure als Prolin steht.

3.  Verfahren nach Anspruch 1 oder 2, **dadurch gekennzeichnet, dass** man eine Interferonvariante der folgenden Formel herstellt:

$$\text{Ala-Pro-}R_0' \qquad\qquad (IV)$$

oder

$$\text{Ala-Gly-}R_0'' \qquad\qquad (V)$$

worin $R_0'$ für die Aminosäuresequenz der reifen Form eines IFN der Trophoblastinklasse steht.

4.  Verfahren nach Anspruch 3, **dadurch gekennzeichnet, dass** man eine Interferonvariante der folgenden Formel herstellt:

$$\text{Ala-Pro-}R_0'' \qquad\qquad (VI)$$

oder

$$\text{Ala-Gly-}R_0'' \qquad\qquad (VII)$$

worin $R_0''$ für die Aminosäuresequenz der reifen Form eines Trophoblastins vom Rind oder Schaf steht.

5.  Verfahren nach Anspruch 4, **dadurch gekennzeichnet, dass** man eine Interferonvariante der folgenden Formel herstellt:

$$\text{Ala-Pro-R}_0''' \tag{VIII}$$

oder

$$\text{Ala-Gly-R}_0''' \tag{IX}$$

worin $R_0'''$ für die reife Form einer der Isoformen des Schaftrophoblastins steht.

6. Verfahren nach Anspruch 5, **dadurch gekennzeichnet, dass** man eine Interferonvariante herstellt, worin $R_0'''$ für eine allgemeine Aminosäuresequenz der Isoformen des Schaftrophoblastins steht, die mit dem Cysteinrest in der Position 1 beginnt und mit dem Prolinrest in der Position 172 endet, worin:

- $R_5$ ein Glutaminsäure-, Glutamin- oder Arginin-Rest ist,

- $R_6$ ein Arginin- oder Lysin-Rest ist,

- $R_{35}$ ein Lysin- oder Asparaginsäure-Rest ist, und

- $R_{44}$ ein Glutaminsäure- oder Asparaginsäure-Rest ist,

- $R_{48}$ ein Leucin- oder Asparaginsäure-Rest ist,

- $R_{49}$ ein Leucin- oder Glutamin-Rest ist.

7. Verfahren nach Anspruch 6, **dadurch gekennzeichnet, dass** man eine Interferonvariante herstellt, die ausgewählt ist aus:

- einer Variante der Formel (Xa) , worin $R_5$ ein Arginin-Rest ist, $R_6$ ein Lysin-Rest ist, $R_{35}$ ein Asparaginsäure-Rest ist, $R_{44}$ ein Glutaminsäure-Rest ist, $R_{48}$ ein Leucin-Rest ist und $R_{49}$ ein Glutamin-Rest ist,

- einer Variante der Formel (Xb), worin $R_5$ ein Glutaminsäure-Rest ist, $R_6$ ein Arginin-Rest ist, $R_{35}$ ein Lysin-Rest ist, $R_{44}$ ein Glutaminsäure-Rest ist, $R_{48}$ ein Asparaginsäure-Rest. ist und $R_{49}$ ein Leucin-Rest ist,

- einer Variante der Formel (Xc), worin $R_5$ ein Glutamin-Rest ist, $R_6$ ein Arginin-Rest ist, $R_{35}$ ein Asparaginsäure-Rest ist und $R_{44}$ ein Asparaginsäure-Rest ist, und

- einer Variante der Formel (Xd) , worin $R_5$ ein Glutamin-Rest ist, $R_6$ ein Arginin-Rest ist, $R_{35}$ ein Asparaginsäure-Rest ist, $R_{44}$ ein Glutaminsäure-Rest ist, $R_{48}$ ein Leucin-Rest ist und $R_{49}$ ein Glutamin-Rest ist.

8. Verfahren zur Herstellung einer Expressionskassette einer Interferonvariante, die so wie in einem der Ansprüche 1 bis 7 definiert ist, **dadurch gekennzeichnet, dass** man eine Expressionskassette herstellt, die mindestens folgendes umfasst:

- ein erstes DNA-Fragment, das für die Variante codiert,

- ein zweites DNA-Fragment, das für ein Peptidsignal codiert, wobei das zweite DNA-Fragment mit dem 5'-Ende des ersten DNA-Fragments verbunden ist,

- einen Promotor, der die Expression der DNA-Fragmente in der Hefe gestattet.

9. Verfahren nach Anspruch 8, **dadurch gekennzeichnet, dass** man eine Expressionskassette herstellt, worin das zweite DNA-Fragment für das Peptidsignal des Vorläufers des Faktors α oder für das Peptidsignal des Vorläufers der β-1,-3- Glucanase der Hefe oder für ein funktionelles Derivat davon codiert.

10. Verfahren nach Anspruch 8 oder Anspruch 9, **dadurch gekennzeichnet, dass** man eine Expressionskassette herstellt, worin der Promotor des Gens MFα1 oder ein funktionelles Derivat davon ist.

11. Verfahren zur Herstellung einer transformierten Hefezelle, **dadurch gekennzeichnet, dass** man die Hefezelle mit einer Expressionskassette nach einem der Ansprüche 8 bis 10 transformiert.

12. Verfahren zur Herstellung einer Interferonvariante nach einem der Ansprüche 1 bis 7, umfassend die Stufe des Züchtens einer transformierten Hefezelle, erhalten nach einem Verfahren nach Anspruch 11 und des Erntens der Variante ausgehend von der Überstehenden Kulturlösung.

13. Verfahren nach Anspruch 12, **dadurch gekennzeichnet, dass** die Variante ausgehend von der Überstehenden Kulturlösung gereinigt wird durch eine Anionenaustauschchromatographie umfassend:

- eine Eluierung durch einen KCL-Gradienten von 0 bis 0,135 M,

- eine isokratische Phase von 0,135 M KCL,

- eine Eluierung durch einen KCL-Gradienten von 0,135 M bis 0,5 M, wobei die Variante zwischen 0,135 M und 0,3 M KCL gesammelt wird.

14. Verfahren zur Herstellung eines Medikaments, **dadurch gekennzeichnet, dass** man eine Interferonvariante nach einem der Ansprüche 1 bis 7verwendet.

15. Verfahren zur Herstellung eines Medikaments, das zu einer therapeutischen Verwendung bestimmt ist, die ausgewählt ist aus:

- einer entzündungshemmenden Verwendung;

- einer antiviralen Verwendung;

- einer antitumoralen Verwendung;

- einer Immunmodulierenden Verwendung;

- einer antiluteolytischen Verwendung,

    **dadurch gekennzeichnet, dass** man eine Interferonvariante nach einem der Ansprüche 1 bis 7 verwendet.

16. Verfahren zur Herstellung einer immunogenen Zusammensetzung, **dadurch gekennzeichnet, dass** man eine Interferonvariante nach einem der Ansprüche 1 bis 7 verwendet.

17. Verfahren zur Herstellung einer Zusammensetzung, die zur Behandlung von Embryonen bei deren Transplantation bestimmt ist, **dadurch gekennzeichnet, dass** man eine Interferonvariante nach einem der Ansprüche 1 bis 7 verwendet.

18. Verfahren zur Herstellung eines diagnostischen Reagenzes, **dadurch gekennzeichnet, dass** man eine Interferonvariante nach einem der Ansprüche 1 bis 7 verwendet.

19. Verfahren zur Herstellung eines Reagenzes, das zur Diagnose der Lebensfähigkeit von Embryonen in ihrem frühen Entwicklungsstadium bestimmt ist, **dadurch gekennzeichnet, dass** man eine Interferonvariante nach einem der Ansprüche 1 bis 7 verwendet.

**Claims**

**Claims for the following Contracting States : AT, BE, CH, DE, DK, FR, GB, IT, LI, LU, NL, SE**

1. A variant interferon (IFN), corresponding to one of the following formulae:

$$X_1\text{-}R_0 \qquad\qquad (I)$$

or alternatively

$$X_1\text{-}X_2\text{-}R_0 \qquad\qquad (II)$$

or alternatively

$$X_1\text{-}X_2\text{-}X_3\text{-}R_0 \qquad\qquad (III)$$

in which $X_1$, $X_2$ and $X_3$ are identical or different and each represent an amino acid,
and $R_0$ represents the amino acid sequence of the mature form of an interferon of the trophoblastin class.

2. A variant as claimed in claim 1, in which $X_1$, $X_2$ and $X_3$ each represent an acidic amino acid, a basic amino acid or an amino acid chosen from the group consisting of alanine, valine, proline, glycine, serine, threonine, cysteine, asparagine and glutamine, on condition that $X_1$ represents an amino acid other than proline.

3. A variant as claimed in either of claims 1 and 2, of formula:

$$\text{Ala-Pro-}R_0' \qquad\qquad (IV)$$

or

$$\text{Ala-Gly-}R_0' \qquad\qquad (V)$$

in which $R_0'$ represents the amino acid sequence of the mature form of an IFN of the trophoblastin class.

4. A variant as claimed in claim 3, of formula:

$$\text{Ala-Pro-}R_0'' \qquad\qquad (VI)$$

or

$$\text{Ala-Gly-}R_0'' \qquad\qquad (VII)$$

in which $R_0''$ represents the amino acid sequence of the mature form of an ovine or bovin trophoblastin.

5. A variant as claimed in claim 4, of formula:

$$\text{Ala-Pro-}R_0''' \qquad\qquad (VIII)$$

or

$$\text{Ala-Gly-}R_0''' \qquad\qquad (IX)$$

in which $R_0'''$ represents the mature form of any one of the isoforms of ovine trophoblastin.

6. A variant as claimed in claim 5, in which $R_0'''$ represents a general amino acid sequence of the isoforms of ovine trophoblastin, beginning with the cysteine residue at position 1 and ending with the proline residue at position 172, in which:

· $R_5$ is a glutamic acid, glutamine or arginine residue,
· $R_6$ is an arginine or lysine residue,
· $R_{35}$ is a lysine or aspartic acid residue,
· $R_{44}$ is a glutamic acid or aspartic acid residue,
· $R_{48}$ is a leucine or aspartic acid residue, and
· $R_{49}$ is a leucine or glutamine residue.

7. A variant as claimed in claim 6, which variant is selected from:

- a variant of formula (Xa) in which $R_5$ is an arginine residue, $R_6$ is a lysine residue, $R_{35}$ is an aspartic acid residue, $R_{44}$ is a glutamic acid residue, $R_{48}$ is a leucine residue and $R_{49}$ a glutamine residue,
- a variant of formula (Xb) in which $R_5$ is a glutamic, acid residue, $R_6$ is an arginine residue, $R_{35}$ is a lysine acid residue, $R_{44}$ is a glutamic acid residue, $R_{48}$ is an aspartic acid residue and $R_{49}$ a leucine residue,
- a variant of formula (Xc) in which $R_5$ is a glutamine residue, $R_6$ is an arginine residue, $R_{35}$ is an aspartic acid residue and $R_{44}$ is an aspartic acid residue, and
- a variant of formula (Xd) in which $R_5$ is a glutamine residue, $R_6$ is an arginine residue, $R_{35}$ is an aspartic acid residue, $R_{44}$ is a glutamic acid residue, $R_{48}$ is a leucine residue and $R_{49}$ a glutamine residue.

8. A cassette for the expression of a variant as claimed in any one of claims 1 to 7, which comprises at least:

- a first DNA fragment coding for said variant,
- a second DNA fragment coding for a signal peptide, said second DNA fragment being linked to the 5' end of the first DNA fragment,
- a promoter enabling said DNA fragments to be expressed in yeast.

9. The expression cassette as claimed in claim 8, in which the second DNA fragment codes for the signal peptide of the precursor of the $\alpha$ factor or for the signal peptide of the precursor of yeast $\beta$-1,3-glucanase or a functional derivative of these peptides.

10. The expression cassette as claimed in either of claims 8 and 9, in which the promoter is that of the MF$\alpha$1 gene or a functional derivative of this promoter.

11. A yeast cell transformed with an expression cassette as claimed in any one of claims 8 to 10.

12. A method for producing a variant as claimed in any one of claims 1 to 7, which comprises the act of culturing a yeast cell as claimed in claim 11 and of harvesting said variant from the culture supernatant.

13. The method as claimed in claim 12, wherein said variant is purified from the culture supernatant by an anion exchange chromatography technique comprising:

- an elution with a KCl gradient from 0 to 0.135 M
- an isocratic phase at 0.135 M KCl
- an elution with a KCl gradient from 0.135 M to 0.5 M,

said variant being collected at between 0.135 M and 0.3 M KCl.

14. An application of a variant of an interferon as claimed in any one of claims 1 to 7, for the production of a medicament.

15. The application of a variant as claimed in any one of claims 1 to 7, for the production of a medicament permitting a therapeutic use selected among:

- an anti-inflammatory use;
- an antiviral use;
- an antitumor use;
- an immunomodulatory use;

- an antiluteolytic use.

16. The application of a variant of an interferon as claimed in any one of claims 1 to 7, for the production of an immunogenic composition.

17. The application of a variant of an interferon as claimed in any one of claims 1 to 7, for the production of a composition permitting a treatment of embryos when they are transplanted.

18. An application of a variant of an interferon as claimed in any one of claims 1 to 7, for the production of a diagnostic reagent.

19. An application of a variant of an interferon as claimed in any one of claims 1 to 7, for the production of a reagent permitting a diagnosis of viability of embryos at an early stage of their development.

**Claims for the following Contracting States : ES, GR**

1. A method for the production of a variant interferon (IFN), comprising producing a variant corresponding to one of the following formulae:

$$X_1\text{-}R_0 \tag{I}$$

or alternatively

$$X_1\text{-}X_2\text{-}R_0 \tag{II}$$

or alternatively

$$X_1\text{-}X_2\text{-}X_3\text{-}R_0 \tag{III}$$

in which $X_1$, $X_2$ and $X_3$ are identical or different and each represent an amino acid,
and $R_0$ represents the amino acid sequence of the mature form of an interferon of the trophoblastin class.

2. A method as claimed in claim 1, comprising producing a variant in which $X_1$, $X_2$ and $X_3$ each represent an acidic amino acid, a basic amino acid or an amino acid chosen from the group consisting of alanine, valine, proline, glycine, serine, threonine, cysteine, asparagine and glutamine, on condition that $X_1$ represents an amino acid other than proline.

3. A method as claimed in either of claims 1 and 2, comprising producing a variant interferon corresponding to one of the following formulae:

$$\text{Ala-Pro-}R_0' \tag{IV}$$

or

$$\text{Ala-Gly-}R_0' \tag{V}$$

in which $R_0'$ represents the amino acid sequence of the mature form of an IFN of the trophoblastin class.

4. A method as claimed in claim 3, comprising producing a variant interferon corresponding to one of the following formulae:

$$\text{Ala-Pro- } R_0'' \qquad\qquad\qquad \text{(VI)}$$

or

$$\text{Ala-Gly- } R_0'' \qquad\qquad\qquad \text{(VII)}$$

in which $R_0''$ represents the amino acid sequence of the mature form of an ovine or bovin trophoblastin.

5. A method as claimed in claim 4, comprising producing a variant interferon corresponding to one of the following formulae:

$$\text{Ala-Pro- } R_0''' \qquad\qquad\qquad \text{(VIII)}$$

or

$$\text{Ala-Gly- } R_0''' \qquad\qquad\qquad \text{(IX)}$$

in which $R_0'''$ represents the mature form of any one of the isoforms of ovine trophoblastin.

6. A method as claimed in claim 5, comprising producing a variant interferon in which $R_0'''$ represents a general amino acid sequence of the isoforms of ovine trophoblastin, beginning with the cysteine residue at position 1 and ending with the proline residue at position 172, in which:

· $R_5$ is a glutamic acid, glutamine or arginine residue,
· $R_6$ is an arginine or lysine residue,
· $R_{35}$ is a lysine or aspartic acid residue,
· $R_{44}$ is a glutamic acid or aspartic acid residue,
· $R_{48}$ is a leucine or aspartic acid residue, and
· $R_{49}$ is a leucine or glutamine residue.

7. A method as claimed in claim 6, comprising producing a variant interferon selected among:

- a variant of formula (Xa) in which $R_5$ is an arginine residue, $R_6$ is a lysine residue, $R_{35}$ is an aspartic acid residue, $R_{44}$ is a glutamic acid residue, $R_{48}$ is a leucine residue and $R_{49}$ a glutamine residue,
- a variant of formula (Xb) in which $R_5$ is a glutamic, acid residue, $R_6$ is an arginine residue, $R_{35}$ is a lysine acid residue, $R_{44}$ is a glutamic acid residue, $R_{48}$ is an aspartic acid residue and $R_{49}$ a leucine residue,
- a variant of formula (Xc) in which $R_5$ is a glutamine residue, $R_6$ is an arginine residue, $R_{35}$ is an aspartic acid residue and $R_{44}$ is an aspartic acid residue, and
- a variant of formula (Xd) in which $R_5$ is a glutamine residue, $R_6$ is an arginine residue, $R_{35}$ is an aspartic acid residue, $R_{44}$ is a glutamic acid residue, $R_{48}$ is a leucine residue and $R_{49}$ a glutamine residue.

8. A method for the production a cassette for the expression of a variant as claimed in any one of claims 1 to 7, comprising producing a cassette for the expression which comprises at least:

- a first DNA fragment coding for said variant,
- a second DNA fragment coding for a signal peptide, said second DNA fragment being linked to the 5' end of the first DNA fragment,
- a promoter enabling said DNA fragments to be expressed in yeast.

9. The method as claimed in claim 8, comprising producing a cassette of expression in which the second DNA fragment codes for the signal peptide of the precursor of the $\alpha$ factor or for the signal peptide of the precursor of yeast $\beta$-1,3-glucanase or a functional derivative of these peptides.

10. The method as claimed in either of claims 8 and 9, comprising producing a cassette of expression in which the promoter is that of the MFα1 gene or a functional derivative of this promoter.

11. A method for the production of a transformed yeast cell, comprising transforming of said yeast cell with an expression cassette as claimed in any one of claims 8 to 10.

12. A method for the production a variant as claimed in any one of claims 1 to 7, which comprises the act of culturing a transformed yeast cell obtained by the process as claimed in claim 11 and of harvesting said variant from the culture supernatant.

13. The method as claimed in claim 12, wherein said variant is purified from the culture supernatant by an anion exchange chromatography technique comprising:

- an elution with a KCl gradient from 0 to 0.135 M
- an isocratic phase at 0.135 M KCl
- an elution with a KCl gradient from 0.135 M to 0.5 M,

said variant being collected at between 0.135 M and 0.3 M KCl.

14. A method for the production of a medicament, comprising using of a variant of an interferon as claimed in any one of claims 1 to 7.

15. A method for the production of a medicament permitting a therapeutic use selected among:

- an anti-inflammatory use;
- an antiviral use;
- an antitumor use;
- an immunomodulatory use;
- an antiluteolytic use;

comprising using a variant of an interferon as claimed in any one of claims 1 to 7.

16. A method for the production of an immunogenic composition, comprising using of a variant of an interferon as claimed in any one of claims 1 to 7.

17. A method for the production of a composition permitting a treatment of embryos when they are transplanted, comprising using of a variant of an interferon as claimed in any one of claims 1 to 7.

18. A method for the production of a diagnostic reagent, comprising using of a variant of an interferon as claimed in any one of claims 1 to 7.

19. A method for the production of a reagent permitting a diagnosis of viability of embryos at an early stage of their development, comprising using of a variant of an interferon as claimed in any one of claims 1 to 7,.

## FIGURE 1

```
ATG GCC TTC GTG CTC TCT CTA CTG ATG GCC CTG GTG CTG GTC      42
Met Ala Phe Val Leu Ser Leu Leu Met Ala Leu Val Leu Val
-23         -20               -15                    -10


AGC TAT GGC CCA GGA GGA TCT CTG GGT TGT TAC CTA TCT CAG      84
Ser Tyr Gly Pro Gly Gly Ser Leu Gly Cys Tyr Leu Ser  R₃
                -5               -1  1                   5


AGA CTC ATG CTG GAT GCC AGG GAG AAC CTC AAG CTC CTG GAC     126
R₆  Leu Met Leu Asp Ala Arg Glu Asn Leu Lys Leu Leu Asp
                     10              15


CGA ATG AAC AGA CTC TCC CCT CAT TCC TGT CTG CAG GAC AGA     168
Arg Met Asn Arg Leu Ser Pro His Ser Cys Leu Gln Asp Arg
20                  25                  30


AAA GAC TTT GGT CTT CCC CAG GAG ATG GTG GAG GGC GAC CAG     210
Lys R₃₅  Phe Gly Leu Pro Gln Glu Met Val R₄₄ Gly Asp Gln
    35                  40                  45


CTC CAG AAG GAC CAG GCC TTC CCT GTG CTC TAC GAG ATG CTC     252
R₄₆ R₄₉  Lys Asp Gln Ala Phe Pro Val Leu Tyr Glu Met Leu
         50                  55                  60


CAG CAG AGC TTC AAC CTC TTC TAC ACA GAG CAC TCC TCT GCT     294
Gln Gln Ser Phe Asn Leu Phe Tyr Thr Glu His Ser Ser Ala
             65                  70                  75


GCC TGG GAC ACC ACC CTC CTG GAC CAG CTC TGC ACT GGA CTC     336
Ala Trp Asp Thr Thr Leu Leu Asp Gln Leu Cys Thr Gly Leu
                 80                  85


CAA CAG CAG CTG GAC CAC CTG GAC ACC TGC AGG GGT CAA GTG     378
Gln Gln Gln Leu Asp His Leu Asp Thr Cys Arg Gly Gln Val
90                  95                  100
```

29

FIGURE 1 (Suite)


ATG GGA GAG GAA GAC TCT GAA CTG GGT AAC ATG GAC CCC ATT    420
Met Gly Glu Glu Asp Ser Glu Leu Gly Asn Met Asp Pro Ile
    105                 110                 115


GTG ACC GTG AAG AAG TAC TTC CAG GGC ATC TAT GAC TAC CTG    462
Val Thr Val Lys Lys Tyr Phe Gln Gly Ile Tyr Asp Tyr Leu
        120                 125                 130


CAA.GAG AAG GGA TAC AGC GAC TGC GCC TGG GAA ATC GTC AGA    504
Gln Glu Lys Gly Tyr Ser Asp Cys Ala Trp Glu Ile Val Arg
            · 135                 140                 145

                                    .

GTC GAG ATG ATG AGA GCC CTC ACT GTA TCA ACC ACC TTG CAA    546
Val Glu Met Met Arg Ala Leu Thr Val Ser Thr Thr Leu Gln
                150                 155


AAA AGG TTA ACA AAG ATG GGT GGA GAT CTG AAC TCA CCT TGA    588
Lys Arg Leu Thr Lys Met Gly Gly Asp Leu Asn Ser Pro·Ter
160                 165                 170

FIG.2

EP 0 513 336 B1

FIG.2

(suite)

FIG.3a

FIG.3b

FIGURE 4

FIGURE 5

FIGURE 6

EP 0 513 336 B1

x10³ cpm

25

20

15

10

5

0

A          B

FIGURE 7

x10³ cpm

35

30

25

20

15

10

5

0

A     B     C

FIGURE 8

FIGURE 9

Poids (g)

FIGURE 9a

Radioactivité (cpm)
x10³

FIGURE 9b

## FIGURE 10